(19) Europäisches Patentamt
European Patent Office
Office européen des brevets

(11) EP 1 266 887 B1

(12) # FASCICULE DE BREVET EUROPEEN

(45) Date de publication et mention de la délivrance du brevet:
**17.08.2005 Bulletin 2005/33**

(21) Numéro de dépôt: **02291463.4**

(22) Date de dépôt: **12.06.2002**

(51) Int Cl.7: **C07D 209/94**, C07D 405/12, C07D 409/12, C07D 401/12, C07D 491/04, A61K 31/407, A61P 35/00

(54) **Dérivés d'indénoindolones, leur procédé de préparation et les compositions pharmaceutiques qui les contiennent**

Indenoindolin Derivate, Verfahren zur Herstellung dieser Derivate und diese enthaltende pharmazeutische Präparate

Indenoindoline derivatives, their process of preparation and pharmaceutical compositions containing them

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **13.06.2001 FR 0107718**

(43) Date de publication de la demande:
**18.12.2002 Bulletin 2002/51**

(73) Titulaire: **Les Laboratoires Servier**
**92200 Neuilly-sur-Seine (FR)**

(72) Inventeurs:
- **Wierzbicki, Michel**
  **78620 L'Etang La Ville (FR)**
- **Boussard, Marie-Francoise**
  **78124 Mareil sur Mauldre (FR)**
- **Rousseau, Anne**
  **91160 Longjumeau (FR)**
- **Atassi, Ghanem**
  **92210 Saint Cloud (FR)**
- **Hickman, John**
  **75017 Paris (FR)**
- **Pierre, Alain**
  **78580 Les Alluets le Roi (FR)**
- **Leonce, Stéphane**
  **78000 Versailles (FR)**
- **Guilbaud, Nicolas**
  **78170 La Celle Saint Cloud (FR)**
- **Kraus-Berthier, Laurence**
  **92700 Colombes (FR)**

(56) Documents cités:
**WO-A-90/15799**

EP 1 266 887 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention concerne de nouveaux dérivés d'indénoindolones, leur procédé de préparation, les compositions pharmaceutiques qui les contiennent ainsi que leur utilisation en tant qu'anticancéreux.

Les besoins de la thérapeutique anticancéreuse exigent le développement constant de nouveaux agents antitumoraux, dans le but d'obtenir des médicaments à la fois plus actifs et mieux tolérés. La demande de brevet WO 90/15 799 décrit des dérivés d'indénoindole, ainsi que leur utilité en tant qu'antioxydants.

Les composés de l'invention, outre le fait qu'ils soient nouveaux, présentent des propriétés antitumorales particulièrement intéressantes.

Plus spécifiquement, la présente invention concerne les composés de formule (I):

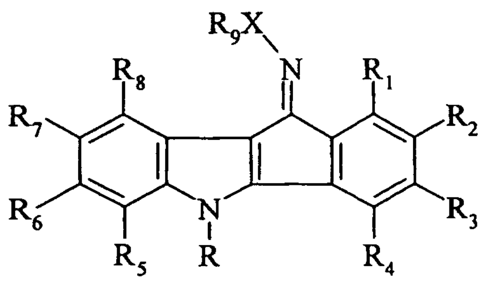

(I)

dans laquelle :

- R représente :

* un atome d'hydrogène,
* un groupement alkyle ($C_1$-$C_6$) linéaire ou ramifié éventuellement substitué par un groupement carboxy, alkoxycarbonyle ($C_1$-$C_6$) linéaire ou ramifié ou $NR_{10}R_{11}$ (dans laquelle $R_{10}$ et $R_{11}$, identiques ou différents, représentent chacun un groupement alkyle ($C_1$-$C_6$) linéaire ou ramifié, ou forment ensemble avec l'atome d'azote qui les porte un hétérocycle azoté),
* ou un groupement alkényle ($C_2$-$C_6$) linéaire ou ramifié,

$R_1$ à $R_8$, identiques ou différents, représentent chacun :

* un atome d'hydrogène,
* un groupement alkyle ($C_1$-$C_6$) linéaire ou ramifié éventuellement substitué par un groupement aryle, carboxy ou alkoxycarbonyle ($C_1$-$C_6$) linéaire ou ramifié,
* un groupement hydroxy,
* un groupement acyloxy ($C_1$-$C_6$) linéaire ou ramifié,
* un groupement de formule $NR_{12}R_{13}$, dans laquelle $R_{12}$ et $R_{13}$, identiques ou différents, représentent chacun un atome d'hydrogène ou un groupement alkyle ($C_1$-$C_6$) linéaire ou ramifié éventuellement substitué par un groupement de formule $NR_{14}R_{15}$ dans laquelle $R_{14}$ et $R_{15}$, identiques ou différents, représentent chacun un groupement alkyle ($C_1$-$C_6$) linéaire ou ramifié, ou forment ensemble avec l'atome d'azote qui les porte un hétérocycle azoté,
* un groupement carboxy,
* un groupement alkoxy ($C_1$-$C_6$) linéaire ou ramifié éventuellement substitué par un groupement aryle ou par un groupement de formule $NR_{14}R_{15}$ dans laquelle $R_{14}$ et $R_{15}$, identiques ou différents, représentent chacun un groupement alkyle ($C_1$-$C_6$) linéaire ou ramifié, ou forment ensemble avec l'atome d'azote qui les porte un hétérocycle azoté,
* un groupement alkényloxy ($C_2$-$C_6$) linéaire ou ramifié,
* ou bien l'un des groupements $R_1$ à $R_8$ forme avec un autre groupement $R_1$ à $R_8$ contigu un groupement alkylènedioxy ($C_1$-$C_2$),

- X représente un atome d'oxygène ou un groupement $NR_{16}$, dans lequel $R_{16}$ représente un atome d'hydrogène ou un groupement alkyle ($C_1$-$C_6$) linéaire ou ramifié, aryle ou arylalkyle ($C_1$-$C_6$) linéaire ou ramifié,

➤ $R_9$ représente un atome d'hydrogène ou un groupement aryle, hétéroaryle ou alkyle ($C_1$-$C_6$) linéaire ou ramifié, étant entendu que le groupement alkyle comporte éventuellement une ou plusieurs insaturations et qu'il est éventuellement substitué par un ou plusieurs groupements, identiques ou différents, choisis parmi aryle, hétéroaryle, cycloalkyle ($C_3$-$C_8$), cyano et $NR_{17}R_{18}$ (dans lequel $R_{17}$ et $R_{18}$, identiques ou différents, représentent chacun un groupement alkyle ($C_1$-$C_6$) linéaire ou ramifié, ou forment ensemble avec l'atome d'azote qui les porte un hétérocycle azoté), leurs isomères ainsi que leurs sels d'addition à un acide ou à une base pharmaceutiquement acceptable.

Par isomères, on entend isomères optiques, et isomères géométriques de la double liaison C = N X $R_9$.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique, camphorique.

Parmi les bases pharmaceutiquement acceptables, on peut citer à titre non limitatif l'hydroxyde de sodium, l'hydroxyde de potassium, la triéthylamine, la tertbutylamine, etc.

Par groupement aryle, on entend phényle, biphénylyle ou naphtyle, chacun de ces groupements étant éventuellement substitué par un ou plusieurs groupements, identiques ou différents, choisis parmi halogène, alkyle ($C_1$-$C_6$) linéaire ou ramifié (éventuellement substitué par un ou plusieurs atomes d'halogène), alkényle ($C_2$-$C_6$) linéaire ou ramifié (éventuellement substitué par un groupement phényle), alkoxy ($C_1$-$C_6$) linéaire ou ramifié (éventuellement substitué par un groupement phényle), phénoxy, nitro, cyano, amino (éventuellement substitué par un ou deux groupements alkyle ($C_1$-$C_6$) linéaire ou ramifié) et alkylènedioxy ($C_1$-$C_2$).

Par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle ($C_1$-$C_6$) linéaire ou ramifié, hydroxy, alkoxy ($C_1$-$C_6$) linéaire ou ramifié, polyhalogénoalkyle ($C_1$-$C_6$) linéaire ou ramifié, et amino (substitué éventuellement par un ou plusieurs groupements alkyle ($C_1$-$C_6$) linéaire ou ramifié). Parmi les groupements hétéroaryle, on peut citer à titre non limitatif les groupements thiényle, pyridyle, furyle, pyrrolyle, imidazolyle, oxazolyle, isoxazolyle, thiazolyle, isothiazolyle, quinolyle, isoquinolyle, pyrimidinyle, thiadiazolyle.
Les groupements hétéroaryle préférés sont les groupements thiényle, pyridyle, furyle et thiadiazolyle.

Par hétérocycle azoté, on entend un groupement monocyclique saturé de 5 à 7 chaînons contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre. Les hétérocycles azotés préférés sont les groupements pyrrolidinyle, pipéridyle, morpholinyle ou pipérazinyle.

Les composés préférés de formule (I) sont ceux pour lesquels X représente un atome d'oxygène.

Les composés préférés de formule (I) sont ceux pour lesquels $R_1$ à $R_6$ et $R_8$, identiques ou différents, représentent chacun un atome d'hydrogène ou un groupement hydroxy ou alkoxy ($C_1$-$C_6$) linéaire ou ramifié.

Les composés préférés de formule (I) sont ceux pour lesquels $R_7$ représente un groupement 2-diméthylaminoéthoxy ou 2-(1-pyrrolidinyl)-éthoxy.

Parmi les composés préférés de formule (I), on peut citer plus particulièrement :

- la (10 Z)-8-(2-diméthylaminoéthoxy)-2,3-diméthoxy-indéno[1,2-b]indol-10(5*H*)-one O-(1-phényl-2-propynyl)oxime, ses isomères ainsi que ses sels d'addition à un acide pharmaceutiquement acceptable,
- la (10 Z)-8-(2-diméthylaminoéthoxy)-2,3-diméthoxy-indéno[1,2-b]indol-10(5*H*)-one O-(2-propynyl)oxime, ses isomères ainsi que ses sels d'addition à un acide pharmaceutiquement acceptable,
- la (10 Z)-8-(2-diméthylaminoéthoxy)-2,3-diméthoxy-indéno[1,2-b]indol-10(5*H*)-one O-(1-méthyl-2-propynyl)oxime, ses isomères ainsi que ses sels d'addition à un acide pharmaceutiquement acceptable,
- la (10 Z)-8-(2-diméthylaminoéthoxy)-2,3-diméthoxy-indéno[1,2-b]indol-10(5H)-one O-[1-(3-furyl)-2-propynyl]oxime, ses isomères ainsi que ses sels d'addition à un acide pharmaceutiquement acceptable,
- la (10 Z)-8-(2-(1-pyrrolidinyl)-éthoxy)-2,3-diméthoxy-indéno[1,2-b]indol-10(5*H*)-one O-[1-(3-furyl)-2-propynyl]oxime, ses isomères ainsi que ses sels d'addition à un acide pharmaceutiquement acceptable,
- et la (10 Z)-8-(2-diméthylaminoéthoxy)-2,3-diméthoxy-indéno[1,2-b]indol-10(5H)-one O-((1S)-1-méthyl-2-propynyl)oxime, ses isomères ainsi que ses sels d'addition à un acide pharmaceutiquement acceptable.

L'invention s'étend également au procédé de préparation des composés de formule (I) caractérisé en ce que l'on met en réaction un composé de formule (II) :

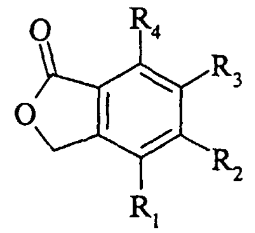

(II)

dans laquelle $R_1$, $R_2$, $R_3$ et $R_4$ ont la même signification que dans la formule (I), avec le N-bromosuccinimide pour conduire au composé de formule (III) :

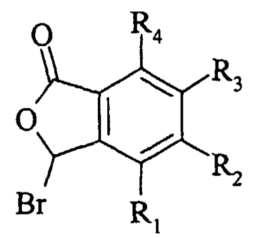

(III)

dans laquelle $R_1$, $R_2$, $R_3$ et $R_4$ ont la même signification que précédemment, que l'on met en réaction avec la triphénylphosphine pour conduire au composé de formule (IV) :

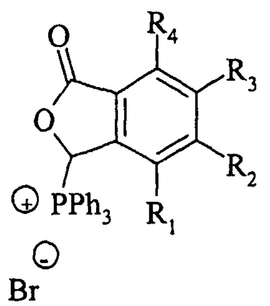

(IV)

dans laquelle $R_1$, $R_2$, $R_3$ et $R_4$ ont la même signification que précédemment, que l'on met en réaction avec un composé de formule (V):

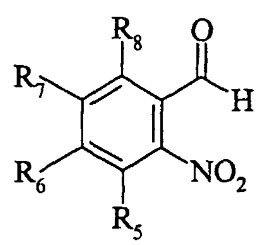

(V)

dans laquelle $R_5$, $R_6$, $R_7$ et $R_8$ ont la même signification que dans la formule (I), pour conduire au composé de formule (VI) :

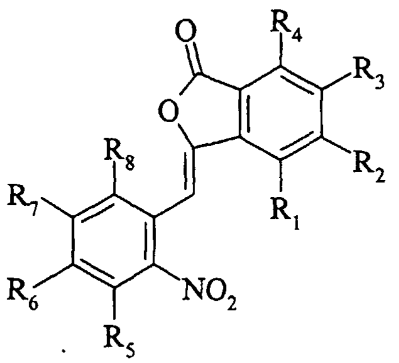

(VI)

dans laquelle $R_1$, $R_2$, $R_3$, $R_4$, $R_5$, $R_6$, $R_7$ et $R_8$ ont la même signification que précédemment, que l'on met en présence de base pour conduire au composé de formule (VII) :

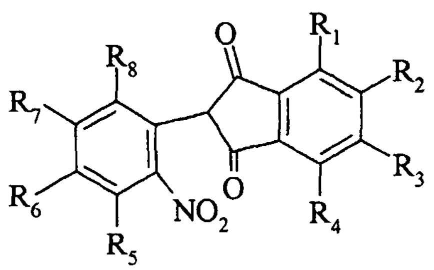

(VII)

dans laquelle $R_1$, $R_2$, $R_3$, $R_4$, $R_5$, $R_6$, $R_7$ et $R_8$ ont la même signification que précédemment, que l'on soumet à un agent de réduction pour conduire, après séparation des isomères le cas échéant, au composé de formule (VIII) :

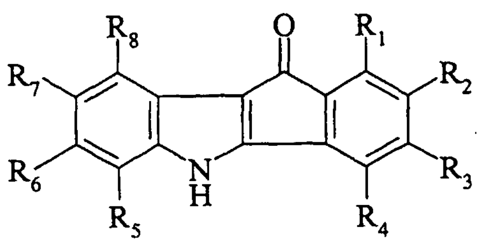

(VIII)

dans laquelle $R_1$, $R_2$, $R_3$, $R_4$, $R_5$, $R_6$, $R_7$ et $R_8$ ont la même signification que précédemment, que l'on met en réaction, lorsqu'on le souhaite, avec un composé de formule (IX) :

$$R'\text{-}Z \qquad (IX)$$

dans laquelle R' représente un groupement alkyle ($C_1$-$C_6$) linéaire ou ramifié, (éventuellement substitué par un groupement aryle ou $NR_9R_{10}$ dans laquelle $R_9$ et $R_{10}$, identiques ou différents, représentent chacun un groupement alkyle ($C_1$-$C_6$) linéaire ou ramifié, ou forment ensemble avec l'atome d'azote qui les porte un hétérocycle azoté) ou alkényle ($C_1$-$C_6$) linéaire ou ramifié, et Z représente un groupement partant tel que, par exemple, un atome d'halogène ou un groupement mésylate, tosylate ou trifluorométhanesulfonate,
pour conduire au composé de formule (X) :

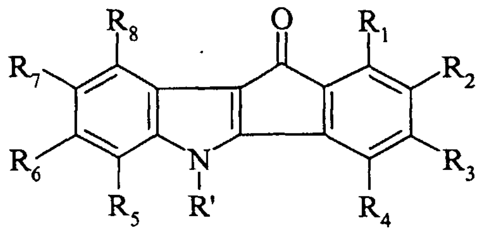

(X)

dans laquelle $R_1$, $R_2$, $R_3$, $R_4$, $R_5$, $R_6$, $R_7$, $R_8$ et R' ont la même signification que précédemment,
composés de formule (VIII) ou (X) que l'on met en réaction avec un composé de formule (XI) :

$$H_2NXR_9 \qquad (XI)$$

dans laquelle X et $R_9$ ont la même signification que dans la formule (I),
pour conduire au composé de formule (I) que l'on purifie, le cas échéant, selon une technique classique de purification, dont on sépare, si on le souhaite, les isomères selon une technique classique de séparation, et que l'on transforme, si on le souhaite, en ses sels d'addition à un acide ou à une base pharmaceutiquement acceptable.

Le composé de formule (XI) peut être obtenu, soit à partir du composé de formule (XII) :

$$R_9\text{-}XH \qquad (XII)$$

dans laquelle X et $R_9$ ont la même signification que précédemment,
selon le procédé décrit dans Synthesis 1976, pp. 682-683 ou dans Synthesis 1980, pp. 461,
soit à partir du composé de formule (XIII):

$$R_9\text{-}Cl \qquad (XIII)$$

dans laquelle $R_9$ est tel que défini précédemment,
selon le procédé décrit dans Tet. Lett. 1997, 38, p. 7233.

Les composés de la présente invention, outre le fait qu'ils soient nouveaux, présentent des propriétés pharmacologiques intéressantes. Ils ont des propriétés cytotoxiques qui les rendent utiles dans le traitement des cancers.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse, intramusculaire ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient et les traitements éventuellement associés. Cette posologie varie de 0,5 mg à 2 g par 24 heures en une ou plusieurs prises.

Les exemples suivants illustrent l'invention et ne la limitent en aucune façon.

Les produits de départ utilisés sont des produits connus ou préparés selon des modes préparatoires connus.

Les préparations A à E conduisent à des intermédiaires de synthèse, utiles pour la préparation des composés de l'invention.

Les structures des composés décrits dans les exemples ont été déterminées selon les techniques spectrométriques usuelles (infrarouge, RMN, spectrométrie de masse).

**Préparation A : 5-(2-Diméthylaminoéthoxy)-2-nitro-benzaldéhyde**

A 20 mmoles de carbonate de potassium en suspension dans un mélange de diméthylformamide et d'éther isopropylique sont additionnées, au goutte à goutte, 10 mmoles de 5-hydroxy-2-nitro-benzaldéhyde en solution dans le diméthylformamide, puis le mélange réactionnel est porté au reflux pendant 2 heures. Après retour à température

ambiante, 10 mmoles de chlorhydrate de 2-chloro-N,N-diméthyléthanamine sont ajoutées au goutte à goutte, puis le mélange réactionnel est à nouveau porté au reflux pendant une nuit. Après retour à température ambiante, le mélange est filtré et les solvants du filtrat sont évaporés pour conduire au produit attendu sous la forme d'une huile.

**Préparation B : 2-Nitro-5-[2-(pipéridin-1-yl)-éthoxy]-benzaldéhyde**

Le produit attendu est obtenu selon le procédé décrit dans la préparation A à partir de 5-hydroxy-2-nitro-benzaldéhyde et de chlorhydrate de 1-(2-chloroéthyl)-pipéridine.

**Préparation C : 2-Nitro-5-(pyrrolidin-1-yl-éthoxy)-benzaldéhyde**

Le produit attendu est obtenu selon le procédé décrit dans la préparation A à partir de 5-hydroxy-2-nitro-benzaldéhyde et de 1-(2-chloroéthyl)-pyrrolidine.

**Préparation D : 5-[N-(2-Diméthylaminoéthyl)-N-méthylamino]-2-nitro-benzaldéhyde**

Le produit attendu est obtenu selon le procédé décrit dans la préparation A à partir de 5-amino-2-nitro-benzaldéhyde et de chlorhydrate de 2-chloro-N,N-diméthyléthanamine.

**Préparation E : 5-(2-Diéthylaminoéthoxy)-2-nitro-benzaldéhyde**

Le produit attendu est obtenu selon le procédé décrit dans la préparation A à partir de 5-hydroxy-2-nitro-benzaldéhyde et de chlorhydrate de 2-chloro-N,N-diéthyléthanamine.

**EXEMPLE 1 : (10 Z)-8-(2-Diméthylaminoéthoxy)-2,3-diméthoxy-indéno[1,2-b]indol -10(5*H*)-one O-(1-phényl-2-propynyl)oxime, chlorhydrate**

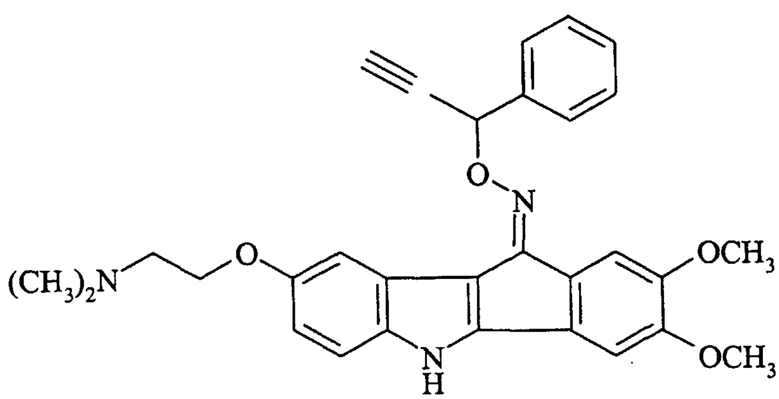

*Stade A : 3-Bromo-5,6-diméthoxy-phtalide*

A 10 mmoles de 5,6-diméthoxy-phtalide en solution dans le dichlorométhane sont ajoutées 12 mmoles de N-bromo-succinimide, puis le mélange réactionnel, éclairé par une lampe halogène, est porté au reflux pendant 2 heures. Le mélange est ensuite ramené à température ambiante et filtré, puis le filtrat est évaporé, du toluène est ajouté, la suspension obtenue est filtrée et le filtrat est évaporé pour conduire au produit attendu.

*Stade B : Bromure de (5,6-diméthoxy-phtalidyl)-triphénylphosphonium*

A 10 mmoles du composé obtenu dans le stade précédent en solution dans le toluène sont ajoutées 10 mmoles de triphénylphosphine, puis le mélange réactionnel est porté au reflux pendant 3 heures. Après retour à température ambiante, le mélange est filtré puis le gâteau obtenu est lavé et séché pour conduire au produit attendu.
*Point de fusion : > 260°C*

*Stade C : 3-[5-(2-Diméthylaminoéthoxy-2-nitro-benzylidène]-5,6-diméthoxy-phtalide*

A 10 mmoles du composé décrit dans la Préparation A en solution dans le diméthylformamide sont ajoutées 10 mmoles de triéthylamine, puis, par portions, 10 mmoles du composé obtenu dans le stade précédent. Le mélange réactionnel est ensuite chauffé à 50°C pendant 1 heure 30, puis ramené à température ambiante et évaporé. De l'éther est ensuite ajouté, le mélange est agité pendant une nuit, puis filtré. Le gâteau obtenu est ensuite lavé pour conduire au produit attendu.

*Stade D : 2-[5-(2-Diméthylaminoéthoxy-2-nitrophényl]-3-hydroxy-5,6-diméthoxy-1H-inden-1-one, chlorhydrate*

A 10 mmoles du composé obtenu dans le stade précédent en solution dans le méthanol sont ajoutés 10 ml d'une solution 4N d'hydroxyde de sodium. Le mélange est ensuite porté à 40°C pendant 1 heure, puis refroidi à 0°C et amené à pH = 1 avec une solution d'acide chlorhydrique 4N (12 ml). Après 3 heures d'agitation à 0°C, le précipité blanc formé est filtré, lavé puis séché pour conduire au produit attendu.
*Point de fusion : 231°C*

*Stade E : 8-(2-Diméthylaminoéthoxy)-2,3-diméthoxy-indéno[1,2-b]indol-10(5H)-one, chlorhydrate*

A 10 mmoles du composé obtenu dans le stade précédent en suspension dans le diméthylformamide sont ajoutées 10 mmoles de triéthylamine. La solution obtenue est ensuite placée sous hydrogène en présence de nickel de Raney. Après filtration du catalyseur, 10 ml d'une solution 1N d'acide chlorhydrique sont ajoutés, puis les solvants sont évaporés. Le résidu obtenu est ensuite repris dans l'éthanol, puis de l'acide chlorhydrique 1N est ajouté. Après filtration, le précipité obtenu est lavé et séché pour conduire au produit attendu.
*Point de fusion : > 260°C*

| *Microanalyse élémentaire :* | | | | |
|---|---|---|---|---|
| | *C%* | *H%* | *N%* | *Cl%* |
| *calculé* | *62,60* | *5,77* | *6,95* | *8,80* |
| *trouvé* | *62,00* | *5,72* | *6,90* | *8,94* |

*Stade F : (10 Z)-8-(2-Diméthylaminoéthoxy)-2,3-diméthoxy-indéno[1,2-b]indol-10(5H)-one O-(1-phényl-2-propynyl) oxime, chlorhydrate*

A 10 mmoles du composé obtenu dans le stade précedent en solution dans le méthanol sont ajoutées 10 mmoles de chlorhydrate de O-(1-phényl-2-propynyl)-hydroxylamine. Après 6 heures d'agitation à température ambiante, le mélange réactionnel est filtré. Le gâteau est lavé puis séché pour conduire au produit attendu.
*Point de fusion : 226°C*

| *Microanalyse élémentaire :* | | | | |
|---|---|---|---|---|
| | *C%* | *H%* | *N%* | *Cl%* |
| *calculé* | *67,73* | *5,68* | *7,90* | *6,66* |
| *trouvé* | *67,82* | *5,74* | *7,97* | *6,76* |

**EXEMPLE 2 : (10 Z)-8-(2-Diméthylaminoéthoxy)-2,3-diméthoxy-indéno[1,2-b]indol -10(5*H*)-one oxime, chlorhydrate**

Le produit attendu est obtenu selon le procédé décrit dans le stade F de l'exemple 1, à partir du composé décrit dans le stade E de l'exemple 1 et de chlorhydrate d'hydroxylamine.
*Point de fusion : > 260°C*

| *Microanalyse élémentaire :* | | | | |
|---|---|---|---|---|
| | *C%* | *H%* | *N%* | *Cl%* |
| *calculé* | *60,36* | *5,79* | *10,06* | *8,48* |
| *trouvé* | *60,35* | *5,77* | *9,97* | *8,52* |

**EXEMPLE 3 : (10 Z)-8-(2-Diméthylaminoéthoxy)-2,3-diméthoxy-indéno[1,2-b]indol -10(5*H*)-one O-méthyloxime, chlorhydrate**

Le produit attendu est obtenu selon le procédé décrit dans le stade F de l'exemple 1, à partir du composé décrit dans le stade E de l'exemple 1 et de chlorhydrate de O-méthyl-hydroxylamine.
*Point de fusion : 250°C*

| *Microanalyse élémentaire :* | | | | |
|---|---|---|---|---|
| | *C%* | *H%* | *N%* | *Cl%* |
| *calculé* | *61,18* | *6,07* | *9,73* | *8,21* |
| *trouvé* | *60,80* | *6,06* | *9,59* | *8,31* |

**EXEMPLE 4 : (10 Z)-8-(2-Diméthylaminoéthoxy)-2,3-diméthoxy-indéno[1,2-b]indol -10(5*H*)-one O-allyloxime, chlorhydrate**

Le produit attendu est obtenu selon le procédé décrit dans le stade F de l'exemple 1, à partir du composé décrit dans le stade E de l'exemple 1 et de chlorhydrate de O-allyl-hydroxylamine.
*Point de fusion : > 260°C*

| *Microanalyse élémentaire :* | | | | |
|---|---|---|---|---|
| | *C%* | *H%* | *N%* | *Cl%* |
| *calculé* | *62,95* | *6,16* | *9,18* | *7,74* |
| *trouvé* | *62,75* | *6,21* | *9,01* | *7,86* |

**EXEMPLE 5 : (10 Z)-8-(2-Diméthylaminoéthoxy)-2,3-diméthoxy-indéno[1,2-b]indol -10(5*H*)-one O-benzyloxime**

Le produit attendu est obtenu selon le procédé décrit dans le stade F de l'exemple 1, à partir du composé décrit dans le stade E de l'exemple 1 et de chlorhydrate de O-benzyl-hydroxylamine.
*Point de fusion : 165°C*

| *Microanalyse élémentaire :* | | | |
|---|---|---|---|
| | *C%* | *H%* | *N%* |
| *calculé* | *71,32* | *6,20* | *8,91* |
| *trouvé* | *71,62* | *6,42* | *8,84* |

**EXEMPLE 6 : (10 Z)-8-(2-Diméthylaminoéthoxy)-2,3-diméthoxy-indéno[1,2-b]indol -10(5*H*)-one O-phényloxime**

Le produit attendu est obtenu selon le procédé décrit dans le stade F de l'exemple 1, à partir du composé décrit dans le stade E de l'exemple 1 et de chlorhydrate de O-phényl-hydroxylamine.
*Point de fusion : 174°C*

| *Microanalyse élémentaire* : | | | |
|---|---|---|---|
| | *C%* | *H%* | *N%* |
| *calculé* | *70,88* | *5,95* | *9,18* |
| *trouvé* | *70,59* | *5,96* | *9,15* |

**EXEMPLE 7 : (10 Z)-8-(2-Diméthylaminoéthoxy)-2,3-diméthoxy-indéno[1,2-b]indol -10(5*H*)-one O-(4-nitrobenzyl)oxime**

Le produit attendu est obtenu selon le procédé décrit dans le stade F de l'exemple 1, à partir du composé décrit dans le stade E de l'exemple 1 et de chlorhydrate de O-(4-nitrobenzyl)-hydroxylamine.
*Point de fusion : 216°C*

| *Microanalyse élémentaire :* | | | |
|---|---|---|---|
| | *C%* | *H%* | *N%* |
| *calculé* | *65,11* | *5,46* | *10,85* |
| *trouvé* | *64,96* | *5,47* | *10,83* |

**EXEMPLE 8 : (10 Z)-8-(2-Diméthylaminoéthoxy)-2,3-diméthoxy-indéno[1,2-b]indol -10(5*H*)-one O-(3-phényl-allyl)oxime, chlorhydrate**

Le produit attendu est obtenu selon le procédé décrit dans le stade F de l'exemple 1, à partir du composé décrit dans le stade E de l'exemple 1 et de chlorhydrate de O-(3-phényl-allyl)-hydroxylamine.
*Point de fusion : 235°C*

| *Microanalyse élémentaire :* | | | | |
|---|---|---|---|---|
| | *C%* | *H%* | *N%* | *Cl%* |
| *calculé* | *67,47* | *6,04* | *7,87* | *6,64* |
| *trouvé* | *67,32* | *6,05* | *7,88* | *6,85* |

**EXEMPLE 9 : (10 Z)-5-Allyl-8-(2-diméthylaminoéthoxy)-2,3-diméthoxy-indéno[1,2-b]indol-10(5*H*)-one O-allyl oxime**

*Stade A : 5-Allyl-8-(2-diméthylaminoéthoxy)-2,3-diméthoxy-indéno[1,2-b]indol-10(5H)-one*

A une solution du composé décrit dans le stade E de l'exemple 1 (10 mmoles) dans le diméthylformamide sont ajoutées 11 mmoles de carbonate de potassium, puis le mélange réactionnel est amené à 90°C et 11 mmoles de bromure d'allyle sont ajoutées. Le milieu réactionnel est ensuite maintenu à 60°C pendant 1 nuit, puis le solvant est évaporé, de l'eau est ajoutée et la suspension obtenue est filtrée. Le gâteau est ensuite recristallisé pour conduire au produit attendu.
*Point de fusion : 138°C*

*Stade B : (10 Z)-5-Allyl-8-(2-diméthylaminoéthoxy)-2,3-diméthoxy-indéno[1,2-b] indol-10(5H)-one O-allyl oxime*

Le produit attendu est obtenu selon le procédé décrit dans le stade F de l'exemple 1, à partir du composé obtenu dans le stade précédent et de chlorhydrate de O-allyl-hydroxylamine.
*Point de fusion : 111°C*

| *Microanalyse élémentaire :* | | | |
|---|---|---|---|
| | *C%* | *H%* | *N%* |
| *calculé* | *70,26* | *6,77* | *9,10* |
| *trouvé* | *70,09* | *6,72* | *9,11* |

**EXEMPLE 10 : (10 Z)-8-(2-Diméthylaminoéthoxy)-2,3-diméthoxy-indéno[1,2-b]indol -10(5*H*)-one O-(3,4-méthylènedioxy-benzyl)oxime**

Le produit attendu est obtenu selon le procédé décrit dans le stade F de l'exemple 1, à partir du composé décrit dans le stade E de l'exemple 1 et de chlorhydrate de O-(3,4-méthylènedioxy-benzyl)-hydroxylamine.
*Point de fusion : 108°C*

| *Microanalyse élémentaire :* | | | |
|---|---|---|---|
| | *C%* | *H%* | *N%* |
| *calculé* | *67,56* | *5,67* | *8,15* |
| *trouvé* | *67,52* | *5,50* | *8,05* |

**EXEMPLE 11 : (10 Z)-8-(2-Diméthylaminoéthoxy)-2,3-diméthoxy-indéno[1,2-b]indol -10(5*H*)-one O-(4-méthoxybenzyl)oxime**

Le produit attendu est obtenu selon le procédé décrit dans le stade F de l'exemple 1, à partir du composé décrit dans le stade E de l'exemple 1 et de chlorhydrate de O-(4-méthoxybenzyl)-hydroxylamine.
*Point de fusion : 220°C*

| *Microanalyse élémentaire :* | | | |
|---|---|---|---|
| | *C%* | *H%* | *N%* |
| *calculé* | *69,44* | *6,23* | *8,38* |
| *trouvé* | *69,15* | *6,26* | *8,33* |

**EXEMPLE 12 : (10 Z)-8-(2-Diméthylaminoéthoxy)-2,3-diméthoxy-indéno[1,2-b]indol -10(5*H*)-one O-(3,5-diméthylbenzyl)oxime**

Le produit attendu est obtenu selon le procédé décrit dans le stade F de l'exemple 1, à partir du composé décrit dans le stade E de l'exemple 1 et de chlorhydrate de O-(3,5-diméthylbenzyl)-hydroxylamine.
*Point de fusion : 181°C*

| *Microanalyse élémentaire :* | | | |
|---|---|---|---|
| | *C%* | *H%* | *N%* |
| *calculé* | *72,12* | *6,66* | *8,41* |
| *trouvé* | *72,16* | *6,71* | *8,35* |

**EXEMPLE 13 : (10 Z)-8-(2-Diméthylaminoéthoxy)-2,3-diméthoxy-indéno[1,2-b]indol -10(5*H*)-one O-(3,4,5-triméthoxybenzyl)oxime**

Le produit attendu est obtenu selon le procédé décrit dans le stade F de l'exemple 1, à partir du composé décrit dans le stade E de l'exemple 1 et de chlorhydrate de O-(3,4,5-triméthoxybenzyl)-hydroxylamine.
*Point de fusion : 213°C*

| *Microanalyse élémentaire :* | | | |
|---|---|---|---|
| | *C%* | *H%* | *N%* |
| *calculé* | *66,30* | *6,28* | *7,48* |
| *trouvé* | *66,62* | *6,32* | *7,39* |

**EXEMPLE 14 : (10 Z)-8-(2-Diméthylaminoéthoxy)-2,3-diméthoxy-indéno[1,2-b]indol -10(5*H*)-one O-(1-naphtylméthyl)oxime, hémichlorhydrate**

Le produit attendu est obtenu selon le procédé décrit dans le stade F de l'exemple 1, à partir du composé décrit dans le stade E de l'exemple 1 et de chlorhydrate de O-(1-naphtyl-méthyl)-hydroxylamine.
*Point de fusion : 147°C*

| *Microanalyse élémentaire :* | | | | |
|---|---|---|---|---|
| | *C%* | *H%* | *N%* | *Cl%* |
| *calculé* | *71,20* | *5,88* | *7,78* | *3,28* |
| *trouvé* | *71,51* | *5,94* | *7,61* | *3,24* |

**EXEMPLE 15 : (10 Z)-8-(2-Diméthylaminoéthoxy)-2,3-diméthoxy-indéno[1,2-b]indol -10(5*H*)-one O-(2-thiénylméthyl)oxime**

Le produit attendu est obtenu selon le procédé décrit dans le stade F de l'exemple 1, à partir du composé

décrit dans le stade E de l'exemple 1 et de chlorhydrate de O-(2-thiényl-méthyl)-hydroxylamine.
*Point de fusion : 170°C*

| *Microanalyse élémentaire :* | | | | |
|---|---|---|---|---|
| | *C%* | *H%* | *N%* | *S%* |
| *calculé* | *65,39* | *5,70* | *8,80* | *6,71* |
| *trouvé* | *65,32* | *5,83* | *8,67* | *6,73* |

**EXEMPLE 16 : (10 Z)-8-(2-Diméthylaminoéthoxy)-2,3-diméthoxy-indéno[1,2-b]indol -10(5*H*)-one O-benzhydryl oxime**

Le produit attendu est obtenu selon le procédé décrit dans le stade F de l'exemple 1, à partir du composé décrit dans le stade E de l'exemple 1 et de chlorhydrate de O-benzhydryl-hydroxylamine.
*Point de fusion : 234°C*

| *Microanalyse élémentaire :* | | | |
|---|---|---|---|
| | *C%* | *H%* | *N%* |
| *calculé* | *74,57* | *6,07* | *7,67* |
| *trouvé* | *73,81* | *6,05* | *7,42* |

**EXEMPLE 17 : (10 Z)-8-(2-Diméthylaminoéthoxy)-2,3-diméthoxy-indéno[1,2-b]indol -10(5*H*)-one O-(3-phényl-2-propynyl)oxime**

Le produit attendu est obtenu selon le procédé décrit dans le stade F de l'exemple 1, à partir du composé décrit dans le stade E de l'exemple 1 et de chlorhydrate de O-(3-phényl-2-propynyl)-hydroxylamine.
*Point de fusion : 200°C*

| *Microanalyse élémentaire :* | | | |
|---|---|---|---|
| | *C%* | *H%* | *N%* |
| *calculé* | *72,71* | *5,90* | *8,48* |
| *trouvé* | *72,79* | *5,94* | *8,47* |

**EXEMPLE 18 : (10 Z)-8-(2-Diméthylaminoéthoxy)-2,3-diméthoxy-indéno[1,2-b]indol -10(5*H*)-one O-(2-propynyl) oxime, chlorhydrate**

Le produit attendu est obtenu selon le procédé décrit dans le stade F de l'exemple 1, à partir du composé décrit dans le stade E de l'exemple 1 et de chlorhydrate de O-(2-propynyl)-hydroxylamine
*Point de fusion : 230°C*

| *Microanalyse élémentaire :* | | | | |
|---|---|---|---|---|
| | *C%* | *H%* | *N%* | *Cl%* |
| *calculé* | *63,22* | *5,75* | *9,22* | *7,78* |
| *trouvé* | *62,75* | *5,84* | *8,97* | *7,96* |

**EXEMPLE 19 : (10 Z)-8-(2-Diméthylaminoéthoxy)-2,3-diméthoxy-indéno[1,2-b]indol -10(5*H*)-one O-(p-tolyl)oxime, chlorhydrate**

Le produit attendu est obtenu selon le procédé décrit dans le stade F de l'exemple 1, à partir du composé décrit dans le stade E de l'exemple 1 et de chlorhydrate de O-(p-tolyl)-hydroxylamine.
*Point de fusion : 190°C*

| *Microanalyse élémentaire :* | | | | |
|---|---|---|---|---|
| | *C%* | *H%* | *N%* | *Cl%* |
| *calculé* | *66,20* | *5,95* | *8,27* | *6,98* |
| *trouvé* | *66,64* | *5,89* | *8,20* | *7,07* |

**EXEMPLE 20 : (10 Z)-8-(2-Diméthylaminoéthoxy)-2,3-diméthoxy-indéno[1,2-b]indol -10(5*H*)-one O-(3,5-diméthoxyphényl)oxime, chlorhydrate**

Le produit attendu est obtenu selon le procédé décrit dans le stade F de l'exemple 1, à partir du composé décrit dans le stade E de l'exemple 1 et de chlorhydrate de O-(3,5-diméthoxyphényl)-hydroxylamine.
*Point de fusion : 155°C*

| *Microanalyse élémentaire :* | | | | |
|---|---|---|---|---|
| | *C%* | *H%* | *N%* | *Cl%* |
| *calculé* | *62,87* | *5,82* | *7,58* | *6,40* |
| *trouvé* | *62,39* | *5,99* | *7,39* | *6,66* |

**EXEMPLE 21 : (10 Z)-8-(2-Diméthylaminoéthoxy)-2,3-diméthoxy-indéno[1,2-b]indol -10(5*H*)-one O-[4-((E)-styryl)-benzyl]oxime, chlorhydrate**

Le produit attendu est obtenu selon le procédé décrit dans le stade F de l'exemple 1, à partir du composé décrit dans le stade E de l'exemple 1 et de chlorhydrate de O-[4-((E)-styryl)-benzyl]-hydroxylamine.
*Point de fusion : > 260°C*

**EXEMPLE 22: (10 Z)-8-(2-Diméthylaminoéthoxy)-2,3-diméthoxy-indéno[1,2-b]indol -10(5*H*)-one O-(4-phénoxybenzyl)oxime**

Le produit attendu est obtenu selon le procédé décrit dans le stade F de l'exemple 1, à partir du composé décrit dans le stade E de l'exemple 1 et de chlorhydrate de O-(4-phénoxybenzyl)-hydroxylamine.
*Point de fusion : 182°C*

| *Microanalyse élémentaire :* | | | |
|---|---|---|---|
| | *C%* | *H%* | *N%* |
| *calculé* | *72,45* | *5,90* | *7,45* |
| *trouvé* | *72,41* | *5,84* | *7,45* |

**EXEMPLE 23 : (10 Z)-8-(2-Diméthylaminoéthoxy)-2,3-diméthoxy-indéno[1,2-b]indol -10(5*H*)-one O-[(biphényl-4-yl)-méthyl]oxime, chlorhydrate**

Le produit attendu est obtenu selon le procédé décrit dans le stade F de l'exemple 1, à partir du composé décrit dans le stade E de l'exemple 1 et de chlorhydrate de O-[(biphényl-4-yl)-méthyl]-hydroxylamine.
*Point de fusion : > 260°C*

| *Microanalyse élémentaire :* | | | | |
|---|---|---|---|---|
| | *C%* | *H%* | *N%* | *Cl%* |
| *calculé* | *69,91* | *5,87* | *7,19* | *6,07* |
| *trouvé* | *69,39* | *5,75* | *7,17* | *6,18* |

**EXEMPLE 24 : (10 Z)-8-(2-Diméthylaminoéthoxy)-2,3-diméthoxy-indéno[1,2-b]indol -10(5*H*)-one O-(4-benzyloxy-3-méthoxybenzyl)oxime, chlorhydrate**

Le produit attendu est obtenu selon le procédé décrit dans le stade F de l'exemple 1, à partir du composé décrit dans le stade E de l'exemple 1 et de chlorhydrate de O-(4 benzyloxy-3-méthoxybenzyl)-hydroxylamine.
*Point de fusion : > 260°C*

| *Microanalyse élémentaire :* | | | | |
|---|---|---|---|---|
| | *C%* | *H%* | *N%* | *Cl%* |
| *calculé* | *67,13* | *5,95* | *6,52* | *5,50* |
| *trouvé* | *66,91* | *5,89* | *6,54* | *5,60* |

**EXEMPLE 25 : (10 Z)-8-(2-Diméthylaminoéthoxy)-2,3-diméthoxy-indéno[1,2-b]indol -10(5*H*)-one O-(2-pyridylméthyl)oxime, chlorhydrate**

Le produit attendu est obtenu selon le procédé décrit dans le stade F de l'exemple 1, à partir du composé décrit dans le stade E de l'exemple 1 et de chlorhydrate de O-(2-pyridylméthyl)-hydroxylamine
*Point de fusion : 235°C*

| *Microanalyse élémentaire :* | | | | |
|---|---|---|---|---|
| | *C%* | *H%* | *N%* | *Cl%* |
| *calculé* | *63,71* | *5,74* | *11,01* | *6,96* |
| *trouvé* | *63,74* | *5,66* | *10,58* | *6,96* |

**EXEMPLE 26: (10 Z)-8-(2-Diméthylaminoéthoxy)-2,3-diméthoxy-indéno[1,2-b]indol -10(5*H*)-one N-phényl hydrazone, chlorhydrate**

Le produit attendu est obtenu selon le procédé décrit dans le stade F de l'exemple 1, à partir du composé décrit dans le stade E de l'exemple 1 et de chlorhydrate de N-phényl-hydrazine.
*Point de fusion : 200°C*

**EXEMPLE 27 : (10 Z)-8-(2-Diméthylaminoéthoxy)-2,3-diméthoxy-indéno[1,2-b]indol -10(5*H*)-one O-[(2E)-5-phényl-2-pentén-4-ynyl]oxime, chlorhydrate**

Le produit attendu est obtenu selon le procédé décrit dans le stade F de l'exemple 1, à partir du composé décrit dans le stade E de l'exemple 1 et de chlorhydrate de O-[(2E)-5-phényl-2-pentén-4-ynyl]-hydroxylamine.
*Point de fusion : >250°C*

| *Microanalyse élémentaire :* | | | | |
|---|---|---|---|---|
| | *C%* | *H%* | *N%* | *Cl%* |
| *calculé* | *67,87* | *5,78* | *7,53* | *6,35* |
| *trouvé* | *68,88* | *5,81* | *7,53* | *6,46* |

**EXEMPLE 28 : (10 Z)-8-(2-Diméthylaminoéthoxy)-2,3-diméthoxy-indéno[1,2-b]indol -10(5*H*)-one N-(4-méthoxyphényl)hydrazone, chlorhydrate**

Le produit attendu est obtenu selon le procédé décrit dans le stade F de l'exemple 1, à partir du composé décrit dans le stade E de l'exemple 1 et de chlorhydrate de N-(4-méthoxyphényl)-hydrazine.
*Point de fusion : 200°C*

| *Microanalyse élémentaire :* | | | | |
|---|---|---|---|---|
| | *C%* | *H%* | *N%* | *Cl%* |
| *calculé* | *60,10* | *5,78* | *10,02* | *12,67* |
| *trouvé* | *60,30* | *5,68* | *9,79* | *12,34* |

**EXEMPLE 29 : (10 Z)-8-(2-Diméthylaminoéthoxy)-2,3-diméthoxy-indéno[1,2-b]indol -10(5*H*)-one O-(4-fluorobenzyl)oxime, chlorhydrate**

Le produit attendu est obtenu selon le procédé décrit dans le stade F de l'exemple 1, à partir du composé décrit dans le stade E de l'exemple 1 et de chlorhydrate de O-(4-fluorobenzyl)-hydroxylamine.
*Point de fusion : 162° C*

**EXEMPLE 30 : 8-(2-Diméthylaminoéthoxy)-2,3-diméthoxy-indéno[1,2-b]indol-10(5*H*)-one O-[bis-(4-fluorophényl)-méthyl]oxime, sesquichlorhydrate**

Le produit attendu est obtenu selon le procédé décrit dans le stade F de l'exemple 1, à partir du composé décrit dans le stade E de l'exemple 1 et de chlorhydrate O-[bis-(4-fluorophényl)-méthyl]-hydroxylamine.

| *Microanalyse élémentaire :* | | | | |
|---|---|---|---|---|
| | *C%* | *H%* | *N%* | *Cl%* |
| *calculé* | *64,05* | *5,10* | *6,59* | *8,16* |
| *trouvé* | *63,38* | *4,80* | *6,72* | *8,33* |

**EXEMPLE 31 : 8-(2-Diméthylaminoéthoxy)-2,3-diméthoxy-indéno[1,2-b]indol-10(5*H*)-one O-(1-phényl-3-butényl)oxime, chlorhydrate**

Le produit attendu est obtenu selon le procédé décrit dans le stade F de l'exemple 1, à partir du composé décrit dans le stade E de l'exemple 1 et de chlorhydrate de O-(1-phényl-3-butényl)-hydroxylamine.

| *Microanalyse élémentaire :* | | | | |
|---|---|---|---|---|
| | *C%* | *H%* | *N%* | *Cl%* |
| *calculé* | *67,94* | *6,25* | *7,67* | *6,47* |
| *trouvé* | *68,06* | *6,13* | *7, 60* | *6,68* |

**EXEMPLE 32 : (10 Z)-8-(2-Diméthylaminoéthoxy)-2,3-diméthoxy-indéno[1,2-b]indol -10(5*H*)-one O-[3-(4-méthoxyphényl)-2-propynyl]oxime, chlorhydrate**

Le produit attendu est obtenu selon le procédé décrit dans le stade F de l'exemple 1, à partir du composé décrit dans le stade E de l'exemple 1 et de chlorhydrate de O-[3-(4-méthoxyphényl)-2-propynyl]-hydroxylamine.
*Point de fusion : 236° C*

| *Microanalyse élémentaire :* | | | | |
|---|---|---|---|---|
| | *C%* | *H%* | *N%* | *Cl%* |
| *calculé* | *66,24* | *5,74* | *7,48* | *6,31* |
| *trouvé* | *65,72* | *5,69* | *7,42* | *5,63* |

**EXEMPLE 33 : (10 Z)-8-(2-Diméthylaminoéthoxy)-2,3-diméthoxy-indéno[1,2-b]indol -10(5*H*)-one O-[1-(2-phényléthynyl)-2-butynyl]oxime, chlorhydrate**

Le produit attendu est obtenu selon le procédé décrit dans le stade F de l'exemple 1, à partir du composé décrit dans le stade E de l'exemple 1 et de chlorhydrate de O-[1-(2-phényléthynyl)-2-butynyl]-hydroxylamine.
*Point de fusion : 185° C*

| *Microanalyse élémentaire :* | | | | |
|---|---|---|---|---|
| | *C%* | *H%* | *N%* | *Cl%* |
| *calculé* | *69,53* | *5,66* | *7,37* | *6,22* |
| *trouvé* | *70,01* | *5,53* | *7,31* | *6,48* |

**EXEMPLE 34 : (10 Z)-8-(2-Diméthylaminoéthoxy)-2,3-diméthoxy-indéno[1,2-b]indol -10(5*H*)-one O-[1-(4-méthoxyphényl)-3-butényl]oxime**

Le produit attendu est obtenu selon le procédé décrit dans le stade F de l'exemple 1, à partir du composé décrit dans le stade E de l'exemple 1 et de chlorhydrate de O-[1-(4-méthoxyphényl)-3-butényl]-hydroxylamine.
*Point de fusion : 190°C*

**EXEMPLE 35 : (10 Z)-8-(2-Diméthylaminoéthoxy)-2,3-diméthoxy-indéno[1,2-b]indol -10(5*H*)-one O-[3-(2-thiényl)-2-propynyl]oxime, chlorhydrate**

Le produit attendu est obtenu selon le procédé décrit dans le stade F de l'exemple 1, à partir du composé décrit dans le stade E de l'exemple 1 et de chlorhydrate de O-[3-(2-thiényl)-2-propynyl]-hydroxylamine.
*Point de fusion : >260°C*

**EXEMPLE 36 : (10 Z)-8-(2-Diméthylaminoéthoxy)-2,3-diméthoxy-indéno[1,2-b]indol -10(5*H*)-one O-[1-((E)-styryl)-3-butényl]oxime**

Le produit attendu est obtenu selon le procédé décrit dans le stade F de l'exemple 1, à partir du composé décrit dans le stade E de l'exemple 1 et de chlorhydrate de O-[1-((E)-styryl)-3-butényl]-hydroxylamine.
*Point de fusion : 176°C*

| *Microanalyse élémentaire :* | | | |
|---|---|---|---|
| | *C%* | *H%* | *N%* |
| *calculé* | *73,72* | *6,56* | *7,82* |
| *trouvé* | *73,34* | *6,58* | *7,52* |

**EXEMPLE 37 : (10 Z)-8-(2-Diméthylaminoéthoxy)-2,3-diméthoxy-indéno[1,2-b]indol -10(5*H*)-one O-(1-phényl-2-butynyl)oxime**

Le produit attendu est obtenu selon le procédé décrit dans le stade F de l'exemple 1, à partir du composé décrit dans le stade E de l'exemple 1 et de chlorhydrate de O-(1-phényl-2-butynyl)-hydroxylamine.
*Point de fusion : 216°C*

| *Microanalyse élémentaire :* | | | |
|---|---|---|---|
| | *C%* | *H%* | *N%* |
| *calculé* | *73,06* | *6,13* | *8,25* |
| *trouvé* | *72,86* | *6, 22* | *8,06* |

**EXEMPLE 38 : (10 Z)-8-(2-Diméthylaminoéthoxy)-2,3-diméthoxy-indéno[1,2-b]indol -10(5*H*)-one O-[1-(p-tolyl)-2-propynyl]oxime**

Le produit attendu est obtenu selon le procédé décrit dans le stade F de l'exemple 1, à partir du composé décrit dans le stade E de l'exemple 1 et de chlorhydrate de O-[1-(p-tolyl)-2-propynyl]-hydroxylamine.
*Point de fusion : 220°C*

| *Microanalyse élémentaire :* | | | |
|---|---|---|---|
| | *C%* | *H%* | *N%* |
| *calculé* | *73,06* | *6,13* | *8,25* |
| *trouvé* | *72,80* | *6,12* | *8,17* |

**EXEMPLE 39 : (10 Z)-8-(2-Diméthylaminoéthoxy)-2,3-diméthoxy-indéno[1,2-b]indol -10(5*H*)-one O-[(1R)-1-phényl-2-propynyl]oxime, chlorhydrate**

Le produit attendu est obtenu selon le procédé décrit dans le stade F de l'exemple 1, à partir du composé décrit dans le stade E de l'exemple 1 et de chlorhydrate de O-[(1R)-1-phényl-2-propynyl]-hydroxylamine.

**EXEMPLE 40 : (10 Z)-8-(2-Diméthylaminoéthoxy)-2,3-diméthoxy-indéno[1,2-b]indol -10(5*H*)-one O-[(1S)-1-phényl-2-propynyl]oxime, chlorhydrate**

Le produit attendu est obtenu selon le procédé décrit dans le stade F de l'exemple 1, à partir du composé décrit dans le stade E de l'exemple 1 et de chlorhydrate de O-[(1S)-1-phényl-2-propynyl]-hydroxylamine.

**EXEMPLE 41 : 8-(2-Diméthylaminoéthoxy)-2,3-diméthoxy-indéno[1,2-b]indol-10(5*H*)-one O-[1-(4-fluorophényl)-2-propynyl]oxime, chlorhydrate**

Le produit attendu est obtenu selon le procédé décrit dans le stade F de l'exemple 1, à partir du composé décrit dans le stade E de l'exemple 1 et de chlorhydrate de O-[1-(4-fluorophényl)-2-propynyl]-hydroxylamine.
*Point de fusion : (mélange Z/E 86/14) : 210°C*

| *Microanalyse élémentaire :* | | | | |
|---|---|---|---|---|
| | *C%* | *H%* | *N%* | *Cl%* |
| *calculé* | *65,95* | *5,33* | *7,69* | *5,75* |
| *trouvé* | *65,14* | *5,28* | *7,53* | *6, 00* |

**EXEMPLE 42 : (10 Z)-8-(2-Diméthylaminoéthoxy)-2,3-diméthoxy-indéno[1,2-b]indol -10(5*H*)-one O-(2-butynyl) oxime, chlorhydrate**

Le produit attendu est obtenu selon le procédé décrit dans le stade F de l'exemple 1, à partir du composé décrit dans le stade E de l'exemple 1 et de chlorhydrate de O-(2-butynyl)-hydroxylamine.
*Point de fusion : 230°C*

| *Microanalyse élémentaire :* | | | | |
|---|---|---|---|---|
| | *C%* | *H%* | *N%* | *Cl%* |
| *calculé* | *63,89* | *6,01* | *8,94* | *6,73* |
| *trouvé* | *63,98* | *5,98* | *8,91* | *6,54* |

**EXEMPLE 43 : (10 Z)-8-(2-Diméthylaminoéthoxy)-2,3-diméthoxy-indéno[1,2-b]indol -10(5*H*)-one O-[1-(3-thiényl)-2-propynyl]oxime, chlorhydrate**

Le produit attendu est obtenu selon le procédé décrit dans le stade F de l'exemple 1, à partir du composé décrit dans le stade E de l'exemple 1 et de chlorhydrate de O-[1-(3-thiényl)-2-propynyl]-hydroxylamine.

**EXEMPLE 44 (10 Z)-8-(2-Diméthylaminoéthoxy)-2,3-diméthoxy-indéno[1,2-b]indol-10(5*H*)-one O-(3-butynyl) oxime**

Le produit attendu est obtenu selon le procédé décrit dans le stade F de l'exemple 1, à partir du composé décrit dans le stade E de l'exemple 1 et de chlorhydrate de O-(3-butynyl)-hydroxylamine.
*Point de fusion : 158°C*

| *Microanalyse élémentaire :* | | | |
|---|---|---|---|
| | *C%* | *H%* | *N%* |
| *calculé* | *69,27* | *6,28* | *9,69* |
| *trouvé* | *68,81* | *6,158* | *9,19* |

**EXEMPLE 45 : (10 Z)-8-(2-Diméthylaminoéthoxy)-2,3-diméthoxy-indéno[1,2-b]indol -10(5*H*)-one O-[1-(2-thiényl)-2-propynyl]oxime, chlorhydrate**

Le produit attendu est obtenu selon le procédé décrit dans le stade F de l'exemple 1, à partir du composé décrit dans le stade E de l'exemple 1 et de chlorhydrate de O-[1-(2-thiényl)-2-propynyl]-hydroxylamine.

**EXEMPLE 46 : (10 Z)-8-(2-Diméthylaminoéthoxy)-2,3-diméthoxy-indéno[1,2-b]indol-10(5*H*)-one O-(1-méthyl-2-propynyl)oxime, chlorhydrate**

Le produit attendu est obtenu selon le procédé décrit dans le stade F de l'exemple 1, à partir du composé décrit dans le stade E de l'exemple 1 et de chlorhydrate de O-(1-méthyl-2-propynyl)-hydroxylamine.
*Point de fusion : 253°C*

| *Microanalyse élémentaire :* | | | |
|---|---|---|---|
| | *C%* | *H%* | *N%* |
| *calculé* | *63,89* | *6,00* | *8,94* |
| *trouvé* | *63,96* | *6,04* | *8,89* |

**EXEMPLE 47 : (10 Z)-8-(2-Diméthylaminoéthoxy)-2,3-diméthoxy-indéno[1,2-b]indol -10(5*H*)-one O-[1-(3-pyridyl)-2-propynyl]oxime, dichlorhydrate**

Le produit attendu est obtenu selon le procédé décrit dans le stade F de l'exemple 1, à partir du composé décrit dans le stade E de l'exemple 1 et de chlorhydrate de O-[1-(3-pyridyl)-2-propynyl]-hydroxylamine.
*Point de fusion : 164°C*

| *Microanalyse élémentaire :* | | | |
|---|---|---|---|
| | *C%* | *H%* | *N%* |
| *calculé* | *61,16* | *5,31* | *9,84* |
| *trouvé* | *61,14* | *5,52* | *9,72* |

**EXEMPLE 48 : (10 Z)-8-(2-Diméthylaminoéthoxy)-2,3-diméthoxy-indéno[1,2-b]indol -10(5*H*)-one O-cyanométhyl oxime, chlorhydrate**

Le produit attendu est obtenu selon le procédé décrit dans le stade F de l'exemple 1, à partir du composé décrit dans le stade E de l'exemple 1 et de chlorhydrate de O-cyanométhyl-hydroxylamine.
*Point de fusion :* 242°C

| *Microanalyse élémentaire :* | | | |
|---|---|---|---|
| | *C%* | *H%* | *N%* |
| *calculé* | *60,45* | *5,51* | *12,26* |
| *trouvé* | *60,48* | *5,54* | *12,07* |

**EXEMPLE 49 : (10 Z)-8-(2-Diméthylaminoéthoxy)-2,3-diméthoxy-indéno[1,2-b]indol -10(5*H*)-one O-(2,3,4,5,6-pentafluorobenzyl)-hydroxylamine.**

Le produit attendu est obtenu selon le procédé décrit dans le stade F de l'exemple 1, à partir du composé

décrit dans le stade E de l'exemple 1 et de chlorhydrate de O-(1-phényl-2-butynyl)-hydroxylamine.
*Point de fusion : 177°C*

| *Microanalyse élémentaire :* | | | |
|---|---|---|---|
| | *C%* | *H%* | *N%* |
| *calculé* | *56,24* | *4,21* | *7,03* |
| *trouvé* | *55,98* | *3,86* | *7,09* |

**EXEMPLE 50 : (10 Z)-8-(2-Diméthylaminoéthoxy)-2,3-diméthoxy-indéno[1,2-b]indol -10(5*H*)-one O-[1-(4-cyanophényl)-2-propynyl]oxime, chlorhydrate**

Le produit attendu est obtenu selon le procédé décrit dans le stade F de l'exemple 1, à partir du composé décrit dans le stade E de l'exemple 1 et de chlorhydrate de O-[1-(4-cyanophényl)-2-propynyl]-hydroxylamine.
*Point de fusion : 183°C*

| *Microanalyse élémentaire :* | | | |
|---|---|---|---|
| | *C %* | *H%* | *N%* |
| *calculé* | *66,84* | *5,25* | *10,06* |
| *trouvé* | *66,60* | *5,05* | *9,90* |

**EXEMPLE 51 : (10 Z)-8-(2-Diméthylaminoéthoxy)-2,3-diméthoxy-indéno[1,2-b]indol -10(5*H*)-one O-[(4-chloro-1,2,3-thiadiazol-5-yl)-méthyl]oxime, chlorhydrate**

Le produit attendu est obtenu selon le procédé décrit dans le stade F de l'exemple 1, à partir du composé décrit dans le stade E de l'exemple 1 et de chlorhydrate de O-[(4-chloro-1,2,3-thiadiazol-5-yl)-méthyl]-hydroxylamine.
*Point de fusion : (mélange Z/E 90/10) : 211°C*

| *Microanalyse élémentaire :* | | | | |
|---|---|---|---|---|
| | *C%* | *H%* | *N%* | *S%* |
| *calculé* | *52,37* | *4,58* | *12,72* | *5,82* |
| *trouvé* | *52,33* | *4,65* | *12,34* | *6,02* |

**EXEMPLE 52 : (10 Z)-8-(2-Diméthylaminoéthoxy)-2,3-diméthoxy-indéno[1,2-b]indol -10(5*H*)-one O-[1-(3-furyl)-2-propynyl]oxime, chlorhydrate**

Le produit attendu est obtenu selon le procédé décrit dans le stade F de l'exemple 1, à partir du composé décrit dans le stade E de l'exemple 1 et de chlorhydrate de O-[1-(3-furyl)-2-propynyl]-hydroxylamine.
*Point de fusion : 225°C*

**EXEMPLE 53 : (10 Z)-2,3-Diméthoxy-5-méthyl-8-[2-(1-pipéridinyl)-éthoxy]-indéno [1,2-b]indol-10(5*H*)-one oxime, chlorhydrate**

*Stade A : 2,3-Diméthoxy-8-[2-(1-pipéridinyl)-éthoxy]-indéno[1,2-b]indol-10(5H)-one, chlorhydrate*

Le produit attendu est obtenu selon le procédé décrit dans les stades C à E de l'exemple 1, à partir du composé décrit dans le stade B de l'exemple 1 et du composé décrit dans la préparation B.
*Point de fusion : >260°C*

*Stade B : 2,3-Diméthoxy-5-méthyl-8-[2-(1-pipéridinyl)-éthoxy]-indéno[1,2-b]indol-10(5H)-one*

Le produit attendu est obtenu selon le procédé décrit dans le stade A de l'exemple 9, à partir du composé décrit dans le stade précédent, en remplaçant le bromure d'allyle par l'iodométhane.
*Point de fusion : 200°C*

*Stade C : (10 Z)-2,3-Diméthoxy-5-méthyl-8-[2-(1-pipéridinyl)-éthoxy]-indéno[1,2-b] indol-10(5H)-one oxime, chlorhydrate*

Le produit attendu est obtenu selon le procédé décrit dans le stade F de l'exemple 1, à partir du composé obtenu dans le stade précédent et de chlorhydrate d'hydroxylamine.
*Point de fusion : 250°C*

| Microanalyse élémentaire : | | | |
|---|---|---|---|
| | C% | H% | N% |
| calculé | 63,76 | 6,21 | 8,92 |
| trouvé | 63,65 | 6,36 | 8,73 |

**EXEMPLE 54 : (10 Z)-2,3-Diméthoxy-8-[2-(1-pyrrolidinyl)-éthoxy]-indéno[1,2-b] indol-10(5*H*)-one O-allyl oxime**

*Stade A : 2,3-Diméthoxy-8-[2-(1-pyrrolidinyl)-éthoxy]-indéno[1,2-b]indol-10(5H)-one, chlorhydrate*

Le produit attendu est obtenu selon le procédé décrit dans les stades C à E de l'exemple 1, à partir du composé décrit dans le stade B de l'exemple 1 et du composé décrit dans la préparation C.
*Point de fusion : 254°C*

*Stade B : (10 Z)-2,3-Diméthoxy-8-[2-(1-pyrrolidinyl)-éthoxy]-indéno[1,2-b]indol-10(5H)-one O-allyl oxime*

Le produit attendu est obtenu selon le procédé décrit dans le stade F de l'exemple 1, à partir du composé obtenu dans le stade précédent et de chlorhydrate de O-allyl-hydroxylamine.
*Point de fusion : 169°C*

| Microanalyse élémentaire : | | | |
|---|---|---|---|
| | C% | H% | N% |
| calculé | 69,78 | 6,53 | 9,39 |
| trouvé | 69,55 | 6,39 | 9,46 |

**EXEMPLE 55 : (10 Z)-2,3-Diméthoxy-8-[2-(1-pyrrolidinyl)-éthoxy]-indéno[1,2-b] indol-10(5*H*)-one O-tert-butyl oxime**

Le produit attendu est obtenu selon le procédé décrit dans le stade F de l'exemple 1, à partir du composé obtenu dans le stade A de l'exemple 54 et de chlorhydrate de O-tert-butyl-hydroxylamine.
*Point de fusion : 197°C*

| Microanalyse élémentaire : | | | |
|---|---|---|---|
| | C% | H% | N% |
| calculé | 69,96 | 7,17 | 9,06 |
| trouvé | 69, 66 | 7,35 | 9,04 |

**EXEMPLE 56 : (10 E)-8-(2-Diméthylaminoéthoxy)-1,4-diméthoxy-indéno[1,2-b]indol -10(5*H*)-one O-benzyl oxime, chlorhydrate**

*Stade A : 8-(2-Diméthylaminoéthoxy)-1,4-diméthoxy-indéno[1,2-b]indol-10(5H)-one, chlorhydrate*

Le produit attendu est obtenu selon le procédé décrit dans les stades A à E de l'exemple 1, à partir de 4,7-diméthoxy-phtalide et du composé décrit dans la préparation A.
*Point de fusion : 230°C*

*Stade B : (10 E)-8-(2-Diméthylaminoéthoxy)-1,4-diméthoxy-indéno[1,2-b]indol-10(5H)-one O-benzyl oxime, chlorhydrate*

Le produit attendu est obtenu selon le procédé décrit dans le stade F de l'exemple 1, à partir du composé obtenu dans le stade précédent et de chlorhydrate de O-benzyl-hydroxylamine.
*Point de fusion : 218°C*

| *Microanalyse élémentaire :* | | | | |
|---|---|---|---|---|
| | *C%* | *H%* | *N%* | *%Cl* |
| *calculé* | *66,20* | *5,95* | *8,27* | *6,98* |
| *trouvé* | *69,66* | *5,90* | *8,17* | *6,97* |

**EXEMPLE 57 : (10 E)-8-(2-Diméthylaminoéthoxy)-1,4-diméthoxy-indéno[1,2-b]indol-10(5*H*)-one O-(1-phényl-2-propynyl) oxime, chlorhydrate**

Le produit attendu est obtenu selon le procédé décrit dans le stade F de l'exemple 1, à partir du composé décrit dans le stade A de l'exemple 56 et de chlorhydrate de O-(1-phényl-2-propynyl)-hydroxylamine.
*Point de fusion : 226°C*

| *Microanalyse élémentaire :* | | | | |
|---|---|---|---|---|
| | *C%* | *H%* | *N%* | *%Cl* |
| *calculé* | *67,73* | *5,68* | *7,90* | *6,66* |
| *trouvé* | *67,43* | *5,65* | *7,89* | *6,83* |

**EXEMPLE 58 : (10 Z)-8-(2-Diméthylaminoéthoxy)-2,3-méthylènedioxy-indéno[1,2-b] indol-10(5*H*)-one O-(2-propynyl) oxime, chlorhydrate**

Le produit attendu est obtenu selon le procédé décrit dans l'exemple 1, à partir de 5,6-méthylènedioxy-phtalide, du composé décrit dans la préparation A et de chlorhydrate de O-(2-propynyl)-hydroxylamine.
*Point de fusion : >260°C*

| *Microanalyse élémentaire :* | | | |
|---|---|---|---|
| | *C%* | *H%* | *N%* |
| *calculé* | *62,80* | *5,04* | *9,55* |
| *trouvé* | *62,33* | *5,13* | *9,45* |

**EXEMPLE 59 : (10 E)-8-(2-Diméthylaminoéthoxy)-1,3-diméthoxy-indéno[1,2-b]indol -10(5*H*)-one O-(2-propynyl) oxime, chlorhydrate**

Le produit attendu est obtenu selon le procédé décrit dans l'exemple 1, à partir de 4,6-diméthoxy-phtalide, du composé décrit dans la préparation A et de chlorhydrate de O-(2-propynyl)-hydroxylamine.
*Point de fusion : 254°C*

| *Microanalyse élémentaire :* | | | |
|---|---|---|---|
| | *C%* | *H%* | *N%* |
| *calculé* | *63,20* | *5,75* | *9,22* |
| *trouvé* | *63,42* | *6,17* | *9,16* |

**EXEMPLE 60 : (10 E)-8-Hydroxy-1,3-diméthoxy-indéno[1,2-b]indol-10(5*H*)-one O-(2-propynyl) oxime, chlorhydrate**

*Stade A : 8-Hydroxy-1,3-diméthoxy-indéno[1,2-b]indol-10(5H)-one, chlorhydrate*

Le produit attendu est obtenu selon le procédé décrit dans les stades A à E de l'exemple 1, à partir de 4,6-diméthoxy-phtalide et de 5-hydroxy-2-nitro-benzaldéhyde.
*Point de fusion : 230°C*

*Stade B : (10 E)-8-Hydroxy-1,3-diméthoxy-indéno[1,2-b]indol-10(5H)-one O-(2-propynyl) oxime, chlorhydrate*

Le produit attendu est obtenu selon le procédé décrit dans le stade F de l'exemple 1, à partir du composé obtenu dans le stade précédent et de chlorhydrate de O-(2-propynyl)-hydroxylamine.
*Point de fusion : 174°C*

| *Microanalyse élémentaire :* | | | |
|---|---|---|---|
| | *C%* | *H%* | *N%* |
| *calculé* | *62,42* | *4,45* | *7,28* |
| *trouvé* | *62,90* | *4,55* | *7,21* |

**EXEMPLE 61 : 5-(2-Diméthylaminoéthyl)-8-hydroxy-1,3-diméthoxy-indéno[1,2-b] indol-10(5*H*)-one O-(1-phényl-2-propynyl) oxime**

*Stade A : 5-(2-Diméthylaminoéthyl)-8-hydroxy-1,3-diméthoxy-indéno[1,2-b]indol-10(5H)-one*

Le produit attendu est obtenu selon le procédé décrit dans le stade A de l'exemple 9, à partir du composé décrit dans le stade A de l'exemple 60, en remplaçant le bromure d'allyle par la 2-chloro-N,N-diméthyléthanamine.

*Stade B : 5-(2-Diméthylaminoéthyl)-8-hydroxy-1,3-diméthoxy-indéno[1,2-b]indol-10(5H)-one O-(1-phényl-2-propynyl) oxime*

Le produit attendu est obtenu selon le procédé décrit dans le stade F de l'exemple 1, à partir du composé obtenu dans le stade précédent et de chlorhydrate de O-(1-phényl-2-propynyl)-hydroxylamine.
*Point de fusion : (Mélange E/Z 92/8) : 128°C*

| *Microanalyse élémentaire :* | | | |
|---|---|---|---|
| | *C%* | *H%* | *N%* |
| *calculé* | *72,71* | *5,90* | *8,48* |
| *trouvé* | *72,36* | *5,98* | *8,19* |

**EXEMPLE 62 : (10 E)-5-(2-Diméthylaminoéthyl)-8-hydroxy-1,3-diméthoxy-indéno [1,2-b]indol-10(5*H*)-one O-allyl oxime**

Le produit attendu est obtenu selon le procédé décrit dans le stade F de l'exemple 1, à partir du composé décrit dans le stade A de l'exemple 61 et de chlorhydrate de O-allyl-hydroxylamine.
*Point de fusion : 212°C*

**EXEMPLE 63 : (10 Z)-2,3-Diméthoxy-8-hydroxy-indéno[1,2-b]indol-10(5*H*)-one O-(2-propynyl) oxime, chlorhydrate**

*Stade A : 8-Hydroxy-2,3-diméthoxy-indéno[1,2-b]indol-10(5H)-one, chlorhydrate*

Le produit attendu est obtenu selon le procédé décrit dans les stades C à E de l'exemple 1, à partir du composé décrit dans le stade B de l'exemple 1 et de 5-hydroxy-2-nitro-benzaldéhyde.

*Stade B : (10 Z)-2,3-Diméthoxy-8-hydroxy-indéno[1,2-b]indol-10(5H)-one O-(2-propynyl) oxime, chlorhydrate*

Le produit attendu est obtenu selon le procédé décrit dans le stade F de l'exemple 1, à partir du composé obtenu au stade précédent et de chlorhydrate de O-(2-propynyl)-hydroxylamine.
*Point de fusion : 156°C*

**EXEMPLE 64 : (10 Z)-2,4-Diméthoxy-8-hydroxy-indéno[1,2-b]indol-10(5*H*)-one O-(2-propynyl) oxime**

Le produit attendu est obtenu selon le procédé décrit dans les stades A à F de l'exemple 1, à partir de 5,7-diméthoxy-phtalide, de 5-hydroxy-2-nitro-benzaldéhyde et de chlorhydrate de O-(2-propynyl)-hydroxylamine.
*Point de fusion : 175°C*

| *Microanalyse élémentaire :* | | | |
|---|---|---|---|
| | *C%* | *H%* | *N%* |
| *calculé* | *68,96* | *4,63* | *8,04* |
| *trouvé* | *68,81* | *4,62* | *7,84* |

**EXEMPLE 65 : (10 E)-1,4-Diméthoxy-8-hydroxy-indéno[1,2-b]indol-10(5*H*)-one O-(2-propynyl) oxime**

Le produit attendu est obtenu selon le procédé décrit dans les stades A à F de l'exemple 1, à partir de 4,7-diméthoxy-phtalide, de 5-hydroxy-2-nitro-benzaldéhyde et de chlorhydrate de O-(2-propynyl)-hydroxylamine.

| *Microanalyse élémentaire :* | | | |
|---|---|---|---|
| | *C%* | *H%* | *N%* |
| *calculé* | *68,96* | *4,63* | *8,04* |
| *trouvé* | *68,31* | *4,72* | *7,82* |

**EXEMPLE 66 : (10 Z)-2,3-Diméthoxy-indéno[1,2-b]indol-10(5*H*)-one O-(2-diméthylaminoéthyl)oxime, chlorhydrate**

*Stade A : 2,3-Diméthoxy-indéno[1,2-b]indol-10(5H)-one, chlorhydrate*

Le produit attendu est obtenu selon le procédé décrit dans les stades C à E de l'exemple 1, à partir du composé décrit dans le stade B de l'exemple 1 et de 2-nitro-benzaldéhyde.

*Stade B : (10 Z)-2,3-Diméthoxy-indéno[1,2-b]indol-10(5H)-one O-(2-diméthylaminoéthyl)oxime, chlorhydrate*

Le produit attendu est obtenu selon le procédé décrit dans le stade F de l'exemple 1, à partir du composé obtenu dans le stade précédent et de chlorhydrate de O-(2-diméthylaminoéthyl)-hydroxylamine.
*Point de fusion : 189°C*

| *Microanalyse élémentaire :* | | | | |
|---|---|---|---|---|
| | *C%* | *H%* | *N%* | *Cl%* |
| *calculé* | *62,76* | *6,02* | *10,46* | *8,82* |
| *trouvé* | *62,87* | *5,97* | *10,37* | *8,90* |

**EXEMPLE 67 : (10 Z)-5-Allyl-2,3-diméthoxy-indéno[1,2-b]indol-10(5*H*)-one O-(2-diméthylaminoéthyl) oxime**

*Stade A : 5-Allyl-2,3-diméthoxy-indéno[1,2-b]indol-10(5H)-one*

Le produit attendu est obtenu selon le procédé décrit dans le stade A de l'exemple 9, à partir du composé décrit dans le stade A de l'exemple 66.

*Stade B : (10 Z)-5-Allyl-2,3-diméthoxy-indéno[1,2-b]indol-10(5H)-one O-(2-diméthylaminoéthyl) oxime*

Le produit attendu est obtenu selon le procédé décrit dans le stade F de l'exemple 1, à partir du composé obtenu dans le stade précédent et de chlorhydrate de O-(2-diméthylaminoéthyl)-hydroxylamine.
*Point de fusion : 111°C*

| *Microanalyse élémentaire :* | | | |
|---|---|---|---|
| | *C%* | *H%* | *N%* |
| *calculé* | *71,09* | *6,71* | *10,36* |
| *trouvé* | *71,18* | *6,86* | *10,17* |

**EXEMPLE 68 : (10 Z)-2,3-Diméthoxy-8-hydroxy-5-méthyl-indéno[1,2-b]indol-10(5*H*)-one O-(2-propynyl)oxime**

Le produit attendu est obtenu selon le procédé décrit dans les stades B et C de l'exemple 53, à partir du composé obtenu au stade A de l'exemple 63 et de chlorhydrate de O-(2-propynyl)-hydroxylamine.
*Point de fusion : 134°C*

| *Microanalyse élémentaire :* | | | |
|---|---|---|---|
| | *C%* | *H%* | *N%* |
| *calculé* | *69,60* | *5,01* | *7,73* |
| *trouvé* | *69,28* | *4,87* | *7,59* |

**EXEMPLE 69 : (10 Z)-8-[N-(2-Diméthylaminoéthyl)-N-méthylamino]-2,3-diméthoxy-indéno[1,2-b]indol-10(5*H*)-one O-(1-phényl-2-propynyl)oxime, dichlorhydrate**

Le produit attendu est obtenu selon le procédé décrit dans les stades C à F de l'exemple 1 à partir du composé obtenu au stade B de l'exemple 1, du composé décrit dans la préparation D et de chlorhydrate de O-(1-phényl-2-propynyl)-hydroxylamine.

**EXEMPLE 70 : (10 Z)-2,3-Diméthoxy-8-hydroxy-5-méthyl-indéno[1,2-b]indol-10(5*H*)-one O-(1-phényl-2-propynyl)oxime**

Le produit attendu est obtenu selon le procédé décrit dans l'exemple 53 à partir du composé décrit au stade B de l'exemple 1, de 5-hydroxy-2-nitrobenzaldéhyde et de chlorhydrate de O-(1-phényl-2-propynyl)-hydroxylamine.
*Point de fusion :* 237°C

| *Microanalyse élémentaire :* | | | |
|---|---|---|---|
| | *C%* | *H%* | *N%* |
| *calculé* | *73,96* | *5,06* | *6,39* |
| *trouvé* | *73,66* | *5,01* | *6,45* |

**EXEMPLE 71 : (10 Z)-8-[N-(2-Diméthylaminoéthyl)-N-méthylamino])-2,3-diméthoxy-indéno[1,2-b]indol-10(5*H*)-one O-(2-propynyl)oxime, dichlorhydrate**

Le produit attendu est obtenu selon le procédé décrit dans les stades C à F de l'exemple 1 à partir du composé obtenu au stade B de l'exemple 1, du composé décrit dans la préparation D et de chlorhydrate de O-(2-propynyl)-hydroxylamine.
*Point de fusion : 155°C*

| *Microanalyse élémentaire :* | | | |
|---|---|---|---|
| | *C%* | *H%* | *N%* |
| *calculé* | *59,41* | *5,98* | *11,08* |

(suite)

| Microanalyse élémentaire : | | | |
|---|---|---|---|
| | C% | H% | N% |
| trouvé | 59,37 | 5,84 | 10,87 |

**EXEMPLE 72 : (10 Z)-8-[N-(2-Diméthylaminoéthyl)-N-méthylamino])-2,3-diméthoxy-indéno[1,2-b]indol-10(5*H*)-one O-(1-(2-thiényl)-2-propynyl)oxime, dichlorhydrate**

Le produit attendu est obtenu selon le procédé décrit dans les stades C à F de l'exemple 1 à partir du composé obtenu au stade B de l'exemple 1, du composé décrit dans la préparation D et de chlorhydrate de O-(1-(2-thiényl)-2-propynyl)-hydroxylamine.
*Point de fusion : 201°C*

| Microanalyse élémentaire : | | | | |
|---|---|---|---|---|
| | C% | H% | N% | S% |
| calculé | 59,28 | 5,49 | 9,54 | 5,46 |
| trouvé | 59,33 | 5,45 | 9,68 | 5,05 |

**EXEMPLE 73 : (10 Z)-5-Méthyl-2,3,8-triméthoxy-indéno[1,2-b]indol-10(5*H*)-one O-(3-butynyl)oxime**

Le produit attendu est obtenu selon le procédé décrit dans l'exemple 53 à partir du composé décrit au stade B de l'exemple 1, de 5-méthoxy-2-nitrobenzaldéhyde et de chlorhydrate de O-(3-butynyl)-hydroxylamine.

| Microanalyse élémentaire : | | | |
|---|---|---|---|
| | C% | H% | N% |
| calculé | 70,75 | 5,68 | 7,17 |
| trouvé | 70,40 | 5,69 | 7,13 |

**EXEMPLE 74 : (10 Z)-5-Méthyl-2,3,8-triméthoxy-indéno[1,2-b]indol-10(5*H*)-one O-(2-propynyl)oxime**

*Stade A : 2,3,8-Triméthoxy-indéno[1,2-b]indol-10(5H)-one, chlorhydrate*

Le produit attendu est obtenu selon le procédé décrit dans les stades C à E de l'exemple 1, à partir du composé décrit dans le stade B de l'exemple 1 et de 5-méthoxy-2-nitro-benzaldéhyde.

*Stade B : (10 Z)-5-Méthyl-2,3,8-triméthoxy-indéno[1,2-b]indol-10(5H)-one O-(2-propynyl)oxime*

Le produit attendu est obtenu selon le procédé décrit dans les stades B et C de l'exemple 53, à partir du composé obtenu au stade précédent et de chlorhydrate de O-(2-propynyl)-hydroxylamine.
*Point de fusion : 199°C*

| Microanalyse élémentaire : | | | |
|---|---|---|---|
| | C% | H% | N% |
| calculé | 70,20 | 5,36 | 7,44 |
| trouvé | 70,38 | 5,39 | 7,37 |

**EXEMPLE 75 : (10 Z)-2,3,8-Triméthoxy-indéno[1,2-b]indol-10(5*H*)-one O-(1-phényl-2-propynyl)oxime**

Le produit attendu est obtenu selon le procédé décrit dans le stade F de l'exemple 1, à partir du composé obtenu au stade A de l'exemple 74 et de chlorhydrate de O-(1-phényl-2-propynyl)-hydroxylamine.
*Point de fusion : 203°C*

| *Microanalyse élémentaire :* | | | |
|---|---|---|---|
| | *C%* | *H%* | *N%* |
| *calculé* | *73,96* | *5,06* | *6,39* |
| *trouvé* | *73,94* | *5,03* | *6,28* |

**EXEMPLE 76 : (10 Z)-5-Méthyl-2,3,8-triméthoxy-indéno[1,2-b]indol-10(5*H*)-one O-(1-phényl-2-propynyl)oxime**

Le produit attendu est obtenu selon les stades B et C de l'exemple 53, à partir du composé obtenu au stade A de l'exemple 74 et de chlorhydrate de O-(1-phényl-2-propynyl)-hydroxylamine.
*Point de fusion :* > 250°C

| *Microanalyse élémentaire :* | | | |
|---|---|---|---|
| | *C%* | *H%* | *N%* |
| *calculé* | *74,32* | *5,35* | *6,19* |
| *trouvé* | *74,05* | *5,36* | *6,21* |

**EXEMPLE 77 : (10 Z)-2,3,8-Triméthoxy-indéno[1,2-b]indol-10(5*H*)-one O-(2-propynyl)oxime**

Le produit attendu est obtenu selon le procédé décrit dans le stade F de l'exemple 1, à partir du composé obtenu au stade A de l'exemple 74 et de chlorhydrate de O-(2-propynyl)-hydroxylamine.
*Point de fusion : > 250°C*

| *Microanalyse élémentaire :* | | | |
|---|---|---|---|
| | *C%* | *H%* | *N%* |
| *calculé* | *69,60* | *5,01* | *7,73* |
| *trouvé* | *68,99* | *5,08* | *7,69* |

**EXEMPLE 78 : (10 Z)-8-(2-Diméthylaminoéthoxy)-2,3-diéthoxy-indéno[1,2-b]indol -10(5*H*)-one O-[1-(3-furyl)-2-propynyl]oxime, chlorhydrate**

*Stade A : Bromure de (5,6-diéthoxy-phtalidyl)-triphénylphosphonium*

Le produit attendu est obtenu selon le procédé décrit dans les stades A et B de l'exemple 1 à partir de 5,6-diéthoxy-phtalide.

*Stade B : 8-(2-Diméthylaminoéthoxy)-2,3-diéthoxy-indéno[1,2-b]indol-10(5H)-one, chlorhydrate*

Le produit attendu est obtenu selon le procédé décrit dans les stades C à E de l'exemple 1 à partir du composé obtenu au stade précédent et du composé décrit dans la préparation A.

*Stade C : (10 Z)-8-(2-Diméthylaminoéthoxy)-2,3-diéthoxy-indéno[1,2-b]indol -10(5H)-one O-[1-(3-furyl)-2-propynyl] oxime, chlorhydrate*

Le produit attendu est obtenu selon le procédé décrit dans le stade F de l'exemple 1 à partir du composé obtenu au stade précédent et de chlorhydrate de O-[1-(3-furyl)-2-propynyl]-hydroxylamine.
*Point de fusion : 225°C*

| *Microanalyse élémentaire :* | | | |
|---|---|---|---|
| | *C%* | *H%* | *N%* |
| *calculé* | *65,51* | *5,86* | *7,64* |
| *trouvé* | *65,51* | *5,88* | *7,64* |

**EXEMPLE 79 : (10 Z)-8-(2-Diéthylaminoéthoxy)-2,3-diéthoxy-indéno[1,2-b]indol -10(5*H*)-one O-[1-(3-furyl)-2-propynyl]oxime, chlorhydrate**

Le produit attendu est obtenu selon le procédé décrit dans les stades C à F de l'exemple 1 à partir du composé obtenu au stade A de l'exemple 78, du composé décrit dans la préparation E et de chlorhydrate de O-[1-(3-furyl)-2-propynyl]-hydroxylamine.
*Point de fusion : 201 °C*

| *Microanalyse élémentaire :* | | | |
|---|---|---|---|
| | *C%* | *H%* | *N%* |
| *calculé* | *66,48* | *6,28* | *7,27* |
| *trouvé* | *66,16* | *6,28* | *7,26* |

**EXEMPLE 80 : (10 Z)-2,3,8-Triméthoxy-indéno[1,2-b]indol-10(5*H*)-one O-(2- diméthylaminoéthyl)oxime, dichlorhydrate**

Le produit attendu est obtenu selon le procédé décrit dans le stade F de l'exemple 1 à partir du composé obtenu au stade A de l'exemple 74 et de chlorhydrate de O-(2-diméthylaminoéthyl)-hydroxylamine.
*Point de fusion : 242°C*

| *Microanalyse élémentaire :* | | | |
|---|---|---|---|
| | *C%* | *H%* | *N%* |
| *calculé* | *56,41* | *5,82* | *8,97* |
| *trouvé* | *57,07* | *5,77* | *8,99* |

**EXEMPLE 81 : (10 Z)-8-(2-Diéthylaminoéthoxy)-2,3-diméthoxy-indéno[1,2-b]indol -10(5*H*)-one O-[1-(3-furyl)-2-propynyl]oxime, chlorhydrate**

Le produit attendu est obtenu selon le procédé décrit dans les stades C à F de l'exemple 1, à partir du composé obtenu au stade B de l'exemple 1, du composé décrit dans la préparation E et de chlorhydrate de O-[1-(3-furyl)-2-propynyl]-hydroxylamine.
*Point de fusion : 238°C*

| *Microanalyse élémentaire :* | | | |
|---|---|---|---|
| | *C%* | *H%* | *N%* |
| *calculé* | *65,51* | *5,86* | *7,64* |
| *trouvé* | *65,22* | *5,97* | *7,77* |

**EXEMPLE 82 : (10 Z)-8-[2-(1-Pyrrolidinyl)-éthoxy]2,3-diméthoxy-indéno[1,2-b] indol-10(5*H*)-one O-[1-(3-furyl)-2-propynyl]oxime, chlorhydrate**

Le produit attendu est obtenu selon le procédé décrit dans les stades C à F de l'exemple 1, à partir du composé obtenu au stade B de l'exemple 1, du composé décrit dans la préparation C et de chlorhydrate de O-[1-(3-furyl)-2-propynyl]-hydroxylamine.
*Point de fusion : 230°C*

| *Microanalyse élémentaire :* | | | |
|---|---|---|---|
| | *C%* | *H%* | *N%* |
| *calculé* | *65,75* | *5,52* | *7,67* |
| *trouvé* | *65,95* | *5,47* | *7,92* |

**EXEMPLE 83 : (10 Z)-8-[2-(1-Pyrrolidinyl)-éthoxy]-2,3-diéthoxy-indéno[1,2-b]indol-10(5*H*)-one O-[1-(3-furyl)-2-propynyl]oxime, chlorhydrate**

Le produit attendu est obtenu selon le procédé décrit dans les stades C à F de l'exemple 1, à partir du composé obtenu au stade A de l'exemple 78, du composé décrit dans la préparation C et de chlorhydrate de O-[1-(3-furyl)-2-propynyl]-hydroxylamine.
*Point de fusion* : 232°C

| *Microanalyse élémentaire :* | | | |
|---|---|---|---|
| | *C%* | *H%* | *N%* |
| *calculé* | *66,72* | *5,95* | *7,29* |
| *trouvé* | *66,29* | *6,06* | *7,21* |

**EXEMPLE 84 : (10 Z)-8-(2-Diméthylaminoéthoxy)-2,3-diméthoxy-5-méthyl-indéno[1,2-b]indol-10(5*H*)-one O-[1-(3-furyl)-2-propynyl]oxime, chlorhydrate**

Le produit attendu est obtenu selon le procédé décrit dans les stades B et C de l'exemple 53, à partir du composé obtenu au stade E de l'exemple 1 et de chlorhydrate de O-[1-(3-furyl)-2-propynyl]-hydroxylamine.
*Point de fusion : 242°C*

| *Microanalyse élémentaire :* | | | |
|---|---|---|---|
| | *C%* | *H%* | *N%* |
| *calculé* | *64,98* | *5,64* | *7,84* |
| *trouvé* | *64,70* | *5,29* | *8,00* |

**EXEMPLE 85 : (10 Z)-8-(2-Diméthylaminoéthoxy)-2,3-diéthoxy-indéno[1,2-b]indol -10(5*H*)-one O-(1-méthyl-2-propynyl)oxime, chlorhydrate**

Le produit attendu est obtenu selon le procédé décrit dans le stade F de l'exemple 1, à partir du composé décrit au stade B de l'exemple 78 et de chlorhydrate de O-(1-méthyl-2-propynyl)-hydroxylamine.
*Point de fusion : 210°C*

| *Microanalyse élémentaire :* | | | |
|---|---|---|---|
| | *C%* | *H%* | *N%* |
| *calculé* | *65,12* | *6,48* | *8,44* |
| *trouvé* | *64,79* | *6,49* | *8,50* |

**EXEMPLE 86 : (10 Z)-8-(2-Diméthylaminoéthoxy)-2,3-éthylènedioxy-indéno[1,2-b] indol-10(5*H*)-one O-[1-(3-furyl)-2-propynyl]oxime, chlorhydrate**

*Stade A : 8-(2-Diméthylaminoéthoxy)-2,3-éthylènedioxy-indéno[1,2-b]indol-10(5H)-one, chlorhydrate*

Le produit attendu est obtenu selon le procédé décrit dans les stades A à E de l'exemple 1, à partir de 5,6-éthylènedioxy-phtalide et du composé décrit dans la préparation A.

*Stade B : (10 Z)-8-(2-Diméthylaminoéthoxy)-2,3-éthylènedioxy-indéno[1,2-b] indol-10(5H)-one O-[1-(3-furyl)-2-propynyl]oxime, chlorhydrate*

Le produit attendu est obtenu selon le procédé décrit dans le stade F de l'exemple 1, à partir du composé obtenu au stade précédent et de chlorhydrate de O-[1-(3-furyl)-2-propynyl]-hydroxylamine.

**EXEMPLE 87 : (10 Z)-8-(2-Diméthylaminoéthoxy)-indéno[1,2-b]indol-10(5*H*)-one O-[1-(3-furyl)-2-propynyl]oxime, chlorhydrate**

*Stade A : 8-(2-Diméthylaminoéthoxy)-indéno[1,2-b]indol-10(5H)-one, chlorhydrate*

Le produit attendu est obtenu selon le procédé décrit dans les stades A à E de l'exemple 1, à partir de phtalide et du composé décrit dans la préparation A.

*Stade B : (10 Z)-8-(2-Diméthylaminoéthoxy)-indéno[1,2-b]indol-10(5H)-one O-[1-(3-furyl)-2-propynyl]oxime, chlorhydrate*

Le produit attendu est obtenu selon le procédé décrit dans le stade F de l'exemple 1, à partir du composé obtenu au stade précédent et de chlorhydrate de O-[1-(3-furyl)-2-propynyl]-hydroxylamine.

**EXEMPLE 88 : (10 Z)-8-(2-Diméthylaminoéthoxy)-2,3-éthylènedioxy-indéno[1,2-b] indol-10(5*H*)-one O-[1-méthyl-2-propynyl]oxime, chlorhydrate**

Le produit attendu est obtenu selon le procédé décrit dans le stade F de l'exemple 1, à partir du composé obtenu au stade A de l'exemple 86 et de chlorhydrate de O-(1-méthyl-2-propynyl)-hydroxylamine.

**EXEMPLE 89 : (10 Z)-8-(2-Diméthylaminoéthoxy)-indéno[1,2-b]indol-10(5*H*)-one O-[1-méthyl-2-propynyl]oxime, chlorhydrate**

Le produit attendu est obtenu selon le procédé décrit dans le stade F de l'exemple 1, à partir du composé obtenu au stade A de l'exemple 87 et de chlorhydrate de O-(1-méthyl-2-propynyl)-hydroxylamine.

**EXEMPLE 90A : (10 Z)-8-(2-Diméthylaminoéthoxy)-2,3-diméthoxy-indéno[1,2-b]indol-10(5*H*)-one O-((1S)-1-méthyl-2-propynyl)oxime, chlorhydrate**

Le produit attendu est obtenu selon le procédé décrit dans le stade F de l'exemple 1, à partir du composé décrit dans le stade E de l'exemple 1 et de chlorhydrate de O-[(1S)-1-méthyl-2-propynyl]-hydroxylamine.

| *Microanalyse élémentaire :* | | | |
|---|---|---|---|
| | *C%* | *H%* | *N%* |
| *calculé* | *63,89* | *6,00* | *8,94* |
| *trouvé* | *63,48* | *5,88* | *8,72* |

**EXEMPLE 90B : (10 Z)-8-(2-Diméthylaminoéthoxy)-2,3-diméthoxy-indéno[1,2-b]indol-10(5*H*)-one O-((1S)-1-méthyl-2-propynyl)oxime, bis-méthanesulfonate**

Le produit attendu est obtenu par retour base du chlorhydrate obtenu à l'exemple 90A, suivi d'une salification par l'acide méthanesulfonique.
*Point de fusion : >260 °C*

| *Microanalyse élémentaire :* | | | | |
|---|---|---|---|---|
| | *C%* | *H%* | *N%* | *S%* |
| *calculé* | *51,83* | *5,64* | *6,72* | *10,25* |
| *trouvé* | *51,79* | *5,69* | *6,68* | *10,41* |

**EXEMPLE 90C : (10 Z)-8-(2-Diméthylaminoéthoxy)-2,3-diméthoxy-indéno[1,2-b]indol-10(5*H*)-one O-((1S)-1-méthyl-2-propynyl)oxime, fumarate**

Le produit attendu est obtenu par retour base du chlorhydrate obtenu à l'exemple 90A, suivi d'une salification par l'acide fumarique.

**EXEMPLE 90D : (10 Z)-8-(2-Diméthylaminoéthoxy)-2,3-diméthoxy-indéno[1,2-b]indol-10(5*H*)-one O-((1S)-1-méthyl-2-propynyl)oxime, tartrate**

Le produit attendu est obtenu par retour base du chlorhydrate obtenu à l'exemple 90A, suivi d'une salification par l'acide tartrique.

**EXEMPLE 91 : (10 Z)-8-(2-Diméthylaminoéthoxy)-2,3-diméthoxy-indéno[1,2-b]indol -10(5*H*)-one O-((1R)-1-méthyl-2-propynyl)oxime, chlorhydrate**

Le produit attendu est obtenu selon le procédé décrit dans le stade F de l'exemple 1, à partir du composé décrit dans le stade E de l'exemple 1 et de chlorhydrate de O-[(1R)-1-méthyl-2-propynyl]-hydroxylamine.

| *Microanalyse élémentaire :* | | | |
|---|---|---|---|
| | *C%* | *H%* | *N%* |
| *calculé* | *63,89* | *6,00* | *8,94* |
| *trouvé* | *63,77* | *5,95* | *8,74* |

**EXEMPLE 92 : (10 Z)-8-(2-Diméthylaminoéthoxy)-2,3-indéno[1,2-b]indol-10(5*H*)-one O-(1-méthyl-2-propynyl) oxime, chlorhydrate**

Le produit attendu est obtenu selon le procédé décrit dans le stade F de l'exemple 1, à partir du composé décrit au stade A de l'exemple 87 et de chlorhydrate de O-(1-méthyl-2-propynyl)-hydroxylamine.
*Point de fusion : 232-233°C*

| *Microanalyse élémentaire :* | | | |
|---|---|---|---|
| | *C%* | *H%* | *N%* |
| *calculé* | *67,39* | *5,90* | *10,25* |
| *trouvé* | *67,15* | *5,62* | *10,39* |

**EXEMPLE 93 : (10 Z)-8-(2-Diméthylaminoéthoxy)-2,3-diméthoxy-indéno[1,2-b]indol -10(5*H*)-one O-((1R)-1-cyclopropyl-2-propynyl)oxime, chlorhydrate**

Le produit attendu est obtenu selon le procédé décrit dans le stade F de l'exemple 1, à partir du composé décrit au stade E de l'exemple 1 et de chlorhydrate de O-(1-cyclopropyl-2-propynyl)-hydroxylamine.
*Point de fusion : 234°C*

| *Microanalyse élémentaire :* | | | |
|---|---|---|---|
| | *C%* | *H%* | *N%* |
| *calculé* | *65,38* | *6,10* | *8,47* |
| *trouvé* | *64,57* | *5,99* | *8,30* |

**EXEMPLE 94 : (10 Z)-8-(2-Diméthylaminoéthoxy)-2,3-indéno[1,2-b]indol-10(5*H*)-one O-(1-cyclopropyl-2-propynyl)oxime, chlorhydrate**

Le produit attendu est obtenu selon le procédé décrit dans le stade F de l'exemple 1, à partir du composé décrit au stade A de l'exemple 87 et de chlorhydrate de O-(1-cyclopropyl-2-propynyl)-hydroxylamine.
*Point de fusion : 225°C*

| *Microanalyse élémentaire :* | | | |
|---|---|---|---|
| | *C%* | *H%* | *N%* |
| *calculé* | *68,88* | *6,01* | *9,64* |
| *trouvé* | *68,46* | *5,89* | *9,51* |

**EXEMPLE 95 : (10 Z)-8-(2-Diméthylaminoéthoxy)-2,3-diméthoxy-indéno[1,2-b]indol -10(5*H*)-one O-(1-pentyl-2-propynyl)oxime, chlorhydrate**

Le produit attendu est obtenu selon le procédé décrit dans le stade F de l'exemple 1, à partir du composé décrit dans le stade E de l'exemple 1 et de chlorhydrate de O-[1-pentyl-2-propynyl]-hydroxylamine.
*Point de fusion : 207°C*

| *Microanalyse élémentaire :* | | | |
|---|---|---|---|
| | *C%* | *H%* | *N%* |
| *calculé* | *66,21* | *6,90* | *7,99* |
| *trouvé* | *66,51* | *6,86* | *8,00* |

**EXEMPLE 96: (10 Z)-8-(2-Diméthylaminoéthoxy)-2,3-diméthoxy-indéno[1,2-b]indol -10(5*H*)-one O-((1S)-1-pentyl-2-propynyl)oxime, chlorhydrate**

Le produit attendu est obtenu selon le procédé décrit dans le stade F de l'exemple 1, à partir du composé décrit dans le stade E de l'exemple 1 et de chlorhydrate de O-[(1S)-1-pentyl-2-propynyl]-hydroxylamine.
Point de fusion : 202°C

| Microanalyse élémentaire : | | | |
|---|---|---|---|
| | *C%* | *H%* | *N%* |
| *calculé* | *66,21* | *6,90* | *7,99* |
| *trouvé* | *66,01* | *6,68* | *8,18* |

**EXEMPLE 97: (10 Z)-8-(2-Diméthylaminoéthoxy)-2,3-diméthoxy-indéno[1,2-b]indol -10(5*H*)-one O-((1R)-1-pentyl-2-propynyl)oxime, chlorhydrate**

Le produit attendu est obtenu selon le procédé décrit dans le stade F de l'exemple 1, à partir du composé décrit dans le stade E de l'exemple 1 et de chlorhydrate de O-[(1R)-1-pentyl-2-propynyl]-hydroxylamine.
*Point de fusion : 202°C*

| *Microanalyse élémentaire :* | | | |
|---|---|---|---|
| | *C%* | *H%* | *N%* |
| *calculé* | *66,21* | *6,90* | *7,99* |
| *trouvé* | *65,66* | *6,83* | *8,33* |

**EXEMPLE 98: (10 Z)-8-(2-Diméthylaminoéthoxy)-2,3-diméthoxy-indéno[1,2-b]indol -10(5*H*)-one O-(1-éthyl-2-propynyl)oxime, chlorhydrate**

Le produit attendu est obtenu selon le procédé décrit dans le stade F de l'exemple 1, à partir du composé décrit dans le stade E de l'exemple 1 et de chlorhydrate de O-[1-éthyl-2-propynyl]-hydroxylamine.
*Point de fusion : 244°C*

| *Microanalyse élémentaire :* | | | |
|---|---|---|---|
| | *C%* | *H%* | *N%* |
| *calculé* | *64,52* | *6,25* | *8,68* |
| *trouvé* | *64,17* | *6,24* | *8,73* |

**EXEMPLE 99: (10 Z)-8-(2-Diméthylaminoéthoxy)-2,3-diméthoxy-indéno[1,2-b]indol -10(5*H*)-one O-(1-isopropyl-2-propynyl)oxime, chlorhydrate**

Le produit attendu est obtenu selon le procédé décrit dans le stade F de l'exemple 1, à partir du composé

décrit dans le stade E de l'exemple 1 et de chlorhydrate de O-[1-isopropyl-2-propynyl]-hydroxylamine.
*Point de fusion : 235°C*

| *Microanalyse élémentaire :* | | | |
|---|---|---|---|
| | *C%* | *H%* | *N%* |
| *calculé* | *65,12* | *6,48* | *8,44* |
| *trouvé* | *65,13* | *6,36* | *8,56* |

**EXEMPLE 100: (10 Z)-8-(2-Diméthylaminoéthoxy)-2,3-diméthoxy-indéno[1,2-b]indol -10(5*H*)-one O-(1-isobutyl-2-propynyl)oxime, chlorhydrate**

Le produit attendu est obtenu selon le procédé décrit dans le stade F de l'exemple 1, à partir du composé décrit dans le stade E de l'exemple 1 et de chlorhydrate de O-[1-isobutyl-2-propynyl]-hydroxylamine.
*Point de fusion : 202°C*

| *Microanalyse élémentaire :* | | | |
|---|---|---|---|
| | *C%* | *H%* | *N%* |
| *calculé* | *65,68* | *6,69* | *8,21* |
| *trouvé* | *65,39* | *6,64* | *8,22* |

**EXEMPLE 101: (10 Z)-8-(2-Diméthylaminoéthoxy)-2,3-diméthoxy-indéno[1,2-b]indol -10(5*H*)-one O-(1-éthynyl-2-propynyl)oxime, chlorhydrate**

Le produit attendu est obtenu selon le procédé décrit dans le stade F de l'exemple 1, à partir du composé décrit dans le stade E de l'exemple 1 et de chlorhydrate de O-[1-éthynyl-2-propynyl]-hydroxylamine.

**EXEMPLE 102: (10 Z)-8-(2-Diméthylaminoéthoxy)-2,3-diméthoxy-indéno[1,2-b]indol -10(5*H*)-one O-trityloxime, chlorhydrate**

*Stade A : Chlorhydrate de O-tritylhydroxylamine*

Le produit attendu est obtenu selon le procédé décrit dans Tet. Lett. 1997, 38, p. 7233, à partir de chlorure de trityle.

*Stade B : (10 Z)-8-(2-Diméthylaminoéthoxy)-2,3-diméthoxy-indéno[1,2-b]indol -10(5H)-one O-trityloxime, chlorhydrate*

Le produit attendu est obtenu selon le procédé décrit dans le stade F de l'exemple 1, à partir du composé décrit dans le stade E de l'exemple 1 et du composé obtenu dans le stade précédent.

**EXEMPLE 103: (10 Z)-8-(2-Diméthylaminoéthoxy)-2,3-diméthoxy-indéno[1,2-b]indol -10(5*H*)-one O-(1,1-diméthyl-2-propynyl)-oxime, chlorhydrate**

Le produit attendu est obtenu selon le procédé décrit dans l'exemple 102, à partir du composé décrit dans le stade E de l'exemple 1 et de 3-chloro-3-méthyl-1-butyne.

**EXEMPLE 104: (10 Z)-8-(2-Diméthylaminoéthoxy)-2,3-diméthoxy-indéno[1,2-b]indol -10(5*H*)-one O-(1-éthyl-1-méthyl-2-propynyl)-oxime, chlorhydrate**

Le produit attendu est obtenu selon le procédé décrit dans l'exemple 102, à partir du composé décrit dans le stade E de l'exemple 1 et de 3-chloro-3-méthyl-1-pentyne, dont la préparation est décrite dans J. Org. Chem. 1955, 20, p.95.

**EXEMPLE 105: (10 Z)-8-(2-Diméthylaminoéthoxy)-2,3-diméthoxy-indéno[1,2-b]indol -10(5*H*)-one O-(1-éthynyl-cyclopentyl)-oxime, chlorhydrate**

Le produit attendu est obtenu selon le procédé décrit dans l'exemple 102, à partir du composé décrit dans le stade E de l'exemple 1 et de 1-chloro-1-éthynylcyclopentane.

**ETUDE PHARMACOLOGIQUE DES COMPOSES DE L'INVENTION**

**EXEMPLE 106 : Cytotoxicité in vitro**

Cinq lignées cellulaires ont été utilisées :

- 2 leucémies murines L1210, et P388.
- 1 carcinome pulmonaire humain, non à petites cellules, A549.
- 1 carcinome colique humain, HT29.
- 1 carcinome épidermoïde humain, A-431.

Les cellules sont cultivées dans du milieu RPMI 1640 complet contenant 10 % de sérum de veau foetal, 2 mM de glutamine, 50 unités/ml de pénicilline, 50 µg/ml de streptomycine et 10 mM d'Hepes, pH = 7,4. Les cellules sont réparties dans des microplaques et exposées aux composés cytotoxiques. Les cellules sont ensuite incubées pendant 4 temps de doublement (2 jours : L1210, P388, ou 4 jours : A549, HT29, A-431). Le nombre de cellules viables est ensuite quantifié par un essai colorimétrique, le microculture tetrazolium assay (Cancer Res. 1987, 47, 939-942).

Les résultats sont exprimés en $IC_{50}$, concentration en produit qui inhibe à 50 % la prolifération des cellules traitées par rapport aux cellules non traitées. A titre d'exemple, les composés des exemples 1 et 18 présentent les $IC_{50}$ mentionnées dans le tableau ci-dessous :

| | $IC_{50}$ nM | | | | |
|---|---|---|---|---|---|
| Composés testés | L1210 | P388 | A549 | HT29 | A-431 |
| Exemple 1 | 77 | 221 | 71 | 248 | 87 |
| Exemple 18 | 104 | 241 | 101 | 181 | 174 |

**EXEMPLE 107 : Action sur le cycle cellulaire (L1210)**

Les cellules L1210 sont incubées pendant 21 heures à 37°C en présence de différentes concentrations en produit testés. Les cellules sont ensuite fixées par de l'éthanol à 70 % (v/v), lavées deux fois dans du PBS et incubées 30 minutes à 20°C dans du PBS contenant 100 µg/ml de RNAse et 50 µg/ml d'iodure de propidium. La répartition des cellules dans les différentes phases du cycle cellulaire est ensuite analysée par cytométrie en flux. Les résultats sont exprimés en pourcentage des cellules accumulées en phase G2+M après 21 heures par rapport au témoin (témoin : 20 %). A titre d'exemple, les composés des exemples 1 et 18 induisent une accumulation de 75 % des cellules en phase G2+M après 21 heures à une concentration de 0,25 µM.

**EXEMPLE 108 : Activité *in vivo* : activité antitumorale des composés sur la leucémie P388**

La lignée P388 (leucémie murine) a été fournie par le National Cancer Institute (Frederick USA). Les cellules tumorales ($10^6$ cellules) ont été inoculées au jour 0 par voie i.p. dans la cavité péritonéale de souris BDF1 femelles (Iffa-Credo, France) pesant de 18 à 20 g (groupes de 6 animaux). Les produits ont été administrés aux jours 1, 5 et 9 aux doses indiquées, par voie i.v.

L'activité antitumorale est exprimée en % de T/C :

$$\% \, T/C = \frac{\text{Temps de survie médian des animaux traités}}{\text{Temps de survie médian des animaux contrôles}} \times 100$$

A titre d'exemple, les composés des exemples 1 et 18 sont très actifs à partir de la dose de 50 mg/kg, ils

doublent la survie des animaux traités. (T/C ≥ 200 %)

**EXEMPLE 109 : Activité *in vivo* : activité antitumorale des composés sur le carcinome colique C38 établi**

Des fragments de carcinome colique C38, pesant environ 30 mg, ont été greffés en sous-cutané au jour 0 sur des souris B6D2F1 (Iffa Credo, France).
Après croissance de la tumeur, les souris ont été séparées en groupes contrôle (18 animaux) et traité (6 à 7 animaux), homogènes quant à la taille tumorale . Les produits ont été administrés par voie i.v. une fois par semaine pendant 3 semaines (aux jours 10, 17 et 24), à leur Dose Maximale Tolérée (MTD), MTD/2 et MTD/4.

Les tumeurs ont été mesurées deux fois par semaine et les volumes tumoraux ont été calculés suivant la formule : Volume ($mm^3$) = longueur (mm) x largeur$^2$ ($mm^2$) / 2.

L'activité antitumorale est exprimée en % de T/C :

$$\% \text{ T/C} = \frac{\text{Vt/V0 médian des animaux traités}}{\text{Vt/V0 médian des animaux contrôles}} \times 100$$

V0 et Vt étant respectivement le volume initial de la tumeur et son volume au temps de mesure t.

La dose optimale est la dose qui donne le T/C le plus bas sans toxicité (mort prématurée ou perte de poids supérieure à 20%).

A titre d'exemple, le composé de l'exemple 18 est très actif à partir de la dose de 25 mg/kg (T/C = 24 %).

**EXEMPLE 110 : Composition pharmaceutique**

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

**1.** Composés de formule (I) :

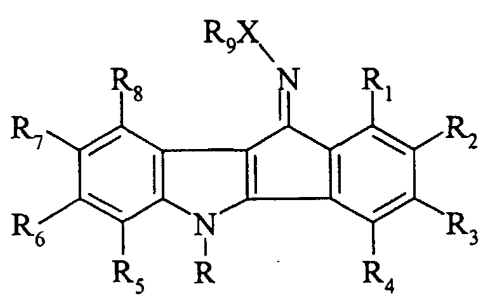

(I)

dans laquelle :

➢ R représente :

* un atome d'hydrogène,
* un groupement alkyle ($C_1$-$C_6$) linéaire ou ramifié éventuellement substitué par un groupement carboxy,

alkoxycarbonyle ($C_1$-$C_6$) linéaire ou ramifié ou $NR_{10}R_{11}$ (dans laquelle $R_{10}$ et $R_{11}$, identiques ou différents, représentent chacun un groupement alkyle ($C_1$-$C_6$) linéaire ou ramifié, ou forment ensemble avec l'atome d'azote qui les porte un hétérocycle azoté),
* ou un groupement alkényle ($C_2$-$C_6$) linéaire ou ramifié,

➢ $R_1$ à $R_8$, identiques ou différents, représentent chacun :

* un atome d'hydrogène,
* un groupement alkyle ($C_1$-$C_6$) linéaire ou ramifié éventuellement substitué par un groupement aryle, carboxy ou alkoxycarbonyle ($C_1$-$C_6$) linéaire ou ramifié,
* un groupement hydroxy,
* un groupement acyloxy ($C_1$-$C_6$) linéaire ou ramifié,
* un groupement de formule $NR_{12}R_{13}$, dans laquelle $R_{12}$ et $R_{13}$, identiques ou différents, représentent chacun un atome d'hydrogène ou un groupement alkyle ($C_1$-$C_6$) linéaire ou ramifié éventuellement substitué par un groupement de formule $NR_{14}R_{15}$ dans laquelle $R_{14}$ et $R_{15}$, identiques ou différents, représentent chacun un groupement alkyle ($C_1$-$C_6$) linéaire ou ramifié, ou forment ensemble avec l'atome d'azote qui les porte un hétérocycle azoté,
* un groupement carboxy,
* un groupement alkoxy ($C_1$-$C_6$) linéaire ou ramifié éventuellement substitué par un groupement aryle ou par un groupement de formule $NR_{14}R_{15}$ dans laquelle $R_{14}$ et $R_{15}$, identiques ou différents, représentent chacun un groupement alkyle ($C_1$-$C_6$) linéaire ou ramifié, ou forment ensemble avec l'atome d'azote qui les porte un hétérocycle azoté,
* un groupement alkényloxy ($C_2$-$C_6$) linéaire ou ramifié,
* ou bien l'un des groupements $R_1$ à $R_8$ forme avec un autre groupement $R_1$ à $R_8$ contigu un groupement alkylènedioxy ($C_1$-$C_2$),

➢ X représente un atome d'oxygène ou un groupement $NR_{16}$, dans lequel $R_{16}$ représente un atome d'hydrogène ou un groupement alkyle ($C_1$-$C_6$) linéaire ou ramifié, aryle ou arylalkyle ($C_1$-$C_6$) linéaire ou ramifié,

➢ $R_9$ représente un atome d'hydrogène ou un groupement aryle, hétéroaryle ou alkyle ($C_1$-$C_6$) linéaire ou ramifié, étant entendu que le groupement alkyle comporte éventuellement une ou plusieurs insaturations et qu'il est éventuellement substitué par un ou plusieurs groupements, identiques ou différents, choisis parmi aryle, hétéroaryle, cycloalkyle ($C_3$-$C_8$), cyano et $NR_{17}R_{18}$ (dans lequel $R_{17}$ et $R_{18}$, identiques ou différents, représentent chacun un groupement alkyle ($C_1$-$C_6$) linéaire ou ramifié, ou forment ensemble avec l'atome d'azote qui les porte un hétérocycle azoté),
leurs isomères ainsi que leurs sels d'addition à un acide ou à une base pharmaceutiquement acceptable, étant entendu que par isomères, on entend isomères optiques, et isomères géométriques de la double liaison $C = N X R_9$,
par groupement aryle, on entend :

phényle, biphénylyle ou naphtyle, chacun de ces groupements étant éventuellement substitué par un ou plusieurs groupements, identiques ou différents, choisis parmi halogène, alkyle ($C_1$-$C_6$)linéaire ou ramifié (éventuellement substitué par un ou plusieurs atomes d'halogène), alkényle ($C_2$-$C_6$) linéaire ou ramifié (éventuellement substitué par un groupement phényle), alkoxy ($C_1$-$C_6$) linéaire ou ramifié (éventuellement substitué par un groupement phényle), phénoxy, nitro, cyano, amino (éventuellement substitué par un ou deux groupements alkyle ($C_1$-$C_6$) linéaire ou ramifié) et alkylènedioxy ($C_1$-$C_2$),

par groupement hétéroaryle, on entend un groupement aromatique mono- ou bicyclique de 5 à 12 chaînons contenant un, deux ou trois hétéroatomes choisis parmi oxygène, azote ou soufre étant entendu que l'hétéroaryle peut être éventuellement substitué par un ou plusieurs groupements, identiques ou différents, choisis parmi halogène, alkyle ($C_1$-$C_6$) linéaire ou ramifié, hydroxy, alkoxy ($C_1$-$C_6$) linéaire ou ramifié, polyhalogénoalkyle (Ci-$C_6$) linéaire ou ramifié, et amino (substitué éventuellement par un ou plusieurs groupements alkyle ($C_1$-$C_6$) linéaire ou ramifié),

et par hétérocycle azoté, on entend un groupement monocyclique saturé de 5 à 7 chaînons contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre.

**2.** Composé de formule (I) selon la revendication 1 tel que X représente un atome d'oxygène.

**3.** Composé de formule (I) selon l'une quelconque des revendications 1 ou 2 tel que $R_1$ à $R_6$ et $R_8$, identiques ou différents, représentent chacun un atome d'hydrogène ou un groupement hydroxy ou alkoxy ($C_1$-$C_6$) linéaire ou ramifié.

**4.** Composé de formule (I) selon l'une quelconque des revendications 1 à 3 tel que $R_7$ représente un groupement 2-diméthylaminoéthoxy ou 2-(1-pyrrolidinyl)-éthoxy.

**5.** Composé de formule (I) selon la revendication 1 qui est la (10 Z)-8-(2-diméthylaminoéthoxy)-2,3-diméthoxy-indéno [1,2-b]indol-10(5*H*)-one O-(1-phényl-2-propynyl)oxime, ses isomères ainsi que ses sels d'addition à un acide pharmaceutiquement acceptable.

**6.** Composé de formule (I) selon la revendication 1 qui est la (10 Z)-8-(2-diméthylaminoéthoxy)-2,3-diméthoxy-indéno [1,2-b]indol-10(5*H*)-one O-(2-propynyl) oxime, ses isomères ainsi que ses sels d'addition à un acide pharmaceutiquement acceptable.

**7.** Composé de formule (I) selon la revendication 1 qui est la (10 Z)-8-(2-diméthylaminoéthoxy)-2,3-diméthoxy-indéno [1,2-b]indol-10(5*H*)-one O-(1-méthyl-2-propynyl) oxime, ses isomères ainsi que ses sels d'addition à un acide pharmaceutiquement acceptable.

**8.** Composé de formule (I) selon la revendication 1 qui est la (10 Z)-8-(2-diméthylaminoéthoxy)-2,3-diméthoxy-indéno [1,2-b]indol-10(5H)-one O-[1-(3-furyl)-2-propynyl]oxime, ses isomères ainsi que ses sels d'addition à un acide pharmaceutiquement acceptable.

**9.** Composé de formule (I) selon la revendication 1 qui est la (10 Z)-8-[2-(1-pyrrolidinyl)-éthoxy]2,3-diméthoxy-indéno [1,2-b]indol-10(5*H*)-one O-[1-(3-furyl)-2-propynyl] oxime, ses isomères ainsi que ses sels d'addition à un acide pharmaceutiquement acceptable.

**10.** Composé de formule (I) selon la revendication 1 qui est la (10 Z)-8-(2-diméthylaminoéthoxy)-2,3-diméthoxy-indéno [1,2-b]indol-10(5H)-one O-((1S)-1-méthyl-2-propynyl) oxime, ses isomères ainsi que ses sels d'addition à un acide pharmaceutiquement acceptable.

**11.** Procédé de préparation des composés de formule (I) selon la revendication 1 **caractérisé en ce que** l'on met en réaction un composé de formule (II) :

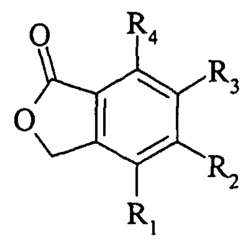

(II)

dans laquelle $R_1$, $R_2$, $R_3$ et $R_4$ ont la même signification que dans la formule (I), avec le N-bromosuccinimide pour conduire au composé de formule (III) :

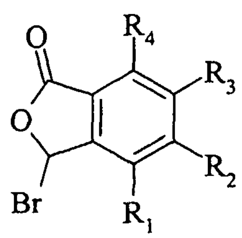

(III)

dans laquelle $R_1$, $R_2$, $R_3$ et $R_4$ ont la même signification que précédemment, que l'on met en réaction avec la triphénylphosphine pour conduire au composé de formule (IV) :

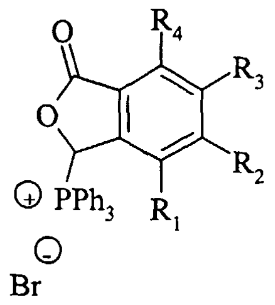

(IV)

dans laquelle $R_1$, $R_2$, $R_3$ et $R_4$ ont la même signification que précédemment, que l'on met en réaction avec un composé de formule (V) :

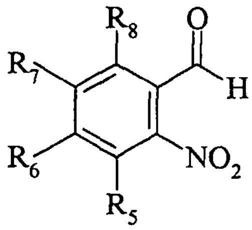

(V)

dans laquelle $R_5$, $R_6$, $R_7$ et $R_8$ ont la même signification que dans la formule (I), pour conduire au composé de formule (VI) :

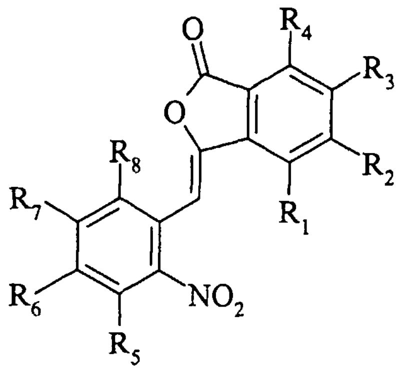

(VI)

dans laquelle $R_1$, $R_2$, $R_3$, $R_4$, $R_5$, $R_6$, $R_7$ et $R_8$ ont la même signification que précédemment, que l'on met en présence de base pour conduire au composé de formule (VII) :

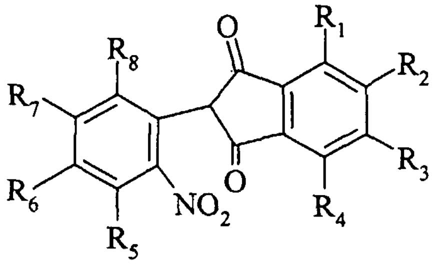

(VII)

dans laquelle $R_1$, $R_2$, $R_3$, $R_4$, $R_5$, $R_6$, $R_7$ et $R_8$ ont la même signification que précédemment, que l'on soumet à un agent de réduction pour conduire, après séparation des isomères le cas échéant, au composé de formule (VIII) :

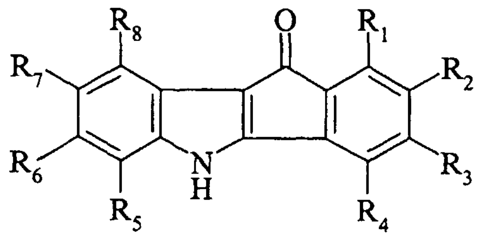

(VIII)

dans laquelle $R_1$, $R_2$, $R_3$, $R_4$, $R_5$, $R_6$, $R_7$ et $R_8$ ont la même signification que précédemment, que l'on met en réaction, lorsqu'on le souhaite, avec un composé de formule (IX) :

$$R'\text{-}Z \qquad \text{(IX)}$$

dans laquelle R' représente un groupement alkyle ($C_1$-$C_6$) linéaire ou ramifié, (éventuellement substitué par un groupement aryle ou $NR_9R_{10}$ dans laquelle $R_9$ et $R_{10}$, identiques ou différents, représentent chacun un groupement alkyle ($C_1$-$C_6$) linéaire ou ramifié, ou forment ensemble avec l'atome d'azote qui les porte un hétérocycle azoté) ou alkényle ($C_1$-$C_6$) linéaire ou ramifié, et Z représente un groupement partant tel que, par exemple, un atome d'halogène ou un groupement mésylate, tosylate ou trifluorométhanesulfonate, pour conduire au composé de formule (X) :

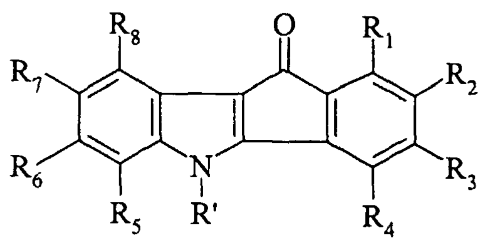

(X)

dans laquelle $R_1$, $R_2$, $R_3$, $R_4$, $R_5$, $R_6$, $R_7$, $R_8$ et R' ont la même signification que précédemment, composés de formule (VIII) ou (X) que l'on met en réaction avec un composé de formule (XI) :

$$H_2NXR_9 \qquad \text{(XI)}$$

dans laquelle X et $R_9$ ont la même signification que dans la formule (I), pour conduire au composé de formule (I) que l'on purifie, le cas échéant, selon une technique classique de purification, dont on sépare, si on le souhaite, les isomères selon une technique classique de séparation, et que l'on transforme, si on le souhaite, en ses sels d'addition à un acide ou à une base pharmaceutiquement acceptable.

**12.** Composition pharmaceutique contenant comme principe actif un composé selon l'une quelconque des revendications 1 à 10, seul ou en combinaison avec un ou plusieurs véhicules inertes, non toxiques et pharmaceutiquement acceptables.

**13.** Composition pharmaceutique selon la revendication 12 utile en tant qu'anticancéreux.

## Claims

**1.** Compounds of formula (I) :

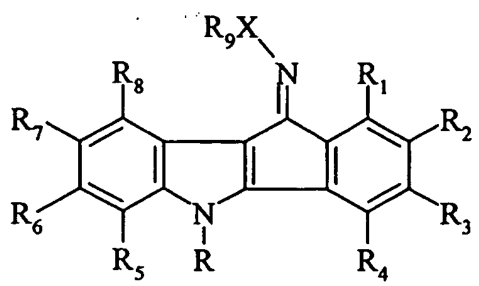

(I)

wherein :

➢ R represents :

* a hydrogen atom,
* a linear or branched ($C_1$-$C_6$)alkyl group optionally substituted by a carboxy group, by a linear or branched ($C_1$-$C_6$)alkoxycarbonyl group or by a $NR_{10}R_{11}$ group (wherein $R_{10}$ and $R_{11}$, which may be identical or different, each represents a linear or branched ($C_1$-$C_6$)alkyl group or together, with the nitrogen carrying them, form a nitrogen-containing heterocycle),
* or a linear or branched ($C_2$-$C_6$)alkenyl group,

➢ $R_1$ to $R_8$, which may be identical or different, each represents :

* a hydrogen atom,
* a linear or branched ($C_1$-$C_6$)alkyl group optionally substituted by an aryl, carboxy or linear or branched ($C_1$-$C_6$)alkoxycarbonyl group,
* a hydroxy group,
* a linear or branched ($C_1$-$C_6$)acyloxy group,
* a group of formula $NR_{12}R_{13}$, wherein $R_{12}$ and $R_{13}$, which may be identical or different, each represents a hydrogen atom or a linear or branched ($C_1$-$C_6$)alkyl group optionally substituted by a group of formula $NR_{14}R_{15}$, wherein $R_{14}$ and $R_{15}$, which may be identical or different, each represents a linear or branched ($C_1$-$C_6$)-alkyl group or together, with the nitrogen atom carrying them, form a nitrogen-containing heterocycle,
* a carboxy group,
* a linear or branched ($C_1$-$C_6$)alkoxy group optionally substituted by an aryl group or by a group of formula $NR_{14}R_{15}$, wherein $R_{14}$ and $R_{15}$, which may be identical or different, each represents a linear or branched ($C_1$-$C_6$)alkyl group or together, with the nitrogen atom carrying them, form a nitrogen-containing heterocycle,
* a linear or branched ($C_2$-$C_6$)alkenyloxy group,
* or one of the groups $R_1$ to $R_8$ forms, with another of the groups $R_1$ to $R_8$ that is adjacent, a ($C_1$-$C_2$) alkylenedioxy group,

➢ X represents an oxygen atom or an $NR_{16}$ group, wherein $R_{16}$ represents a hydrogen atom or a linear or branched ($C_1$-$C_6$)alkyl group, an aryl group or an aryl-($C_1$-$C_6$)alkyl group in which the alkyl moiety may be linear or branched,

➢ $R_9$ represents a hydrogen atom or an aryl, heteroaryl, or linear or branched ($C_1$-$C_6$)alkyl group, wherein the alkyl group optionally contains one or more unsaturated bonds and is optionally substituted by one or more identical or different groups selected from aryl, heteroaryl, ($C_3$-$C_8$)cycloalkyl, cyano and $NR_{17}R_{18}$ (wherein $R_{17}$ and $R_{18}$, which may be identical or different, each represents a linear or branched ($C_1$-$C_6$)alkyl group or together, with the nitrogen atom carrying them, form a nitrogen-containing heterocycle),

isomers thereof, and also addition salts thereof with a pharmaceutically acceptable acid or base,
isomers being understood to mean optical isomers, and geometric isomers of the double bond $C=NXR_9$,
wherein an aryl group is to be understood as:

phenyl, biphenylyl or naphthyl, each of those groups optionally being substituted by one or more identical or different groups selected from halogen, linear or branched $(C_1-C_6)$alkyl (optionally substituted by one or more halogen atoms), linear or branched $(C_2-C_6)$alkenyl (optionally substituted by a phenyl group), linear or branched $(C_1-C_6)$alkoxy (optionally substituted by a phenyl group), phenoxy, nitro, cyano, amino (optionally substituted by one or two linear or branched $(C_1-C_6)$alkyl groups) and $(C_1-C_2)$alkylenedioxy,

a heteroaryl group is to be understood as an aromatic mono- or bi-cyclic group having from 5 to 12 ring members containing one, two or three hetero atoms selected from oxygen, nitrogen and sulphur, wherein the heteroaryl may optionally be substituted by one or more identical or different groups selected from halogen, linear or branched $(C_1-C_6)$-alkyl, hydroxy, linear or branched $(C_1-C_6)$alkoxy, linear or branched $(C_1-C_6)$polyhaloalkyl, and amino (optionally substituted by one or more linear or branched $(C_1-C_6)$alkyl groups),

and a nitrogen-containing heterocycle is to be understood as a saturated monocyclic group having from 5 to 7 ring members containing one, two or three hetero atoms, one of those hetero atoms being a nitrogen atom, and the additional hetero atom or atoms optionally present being selected from the atoms oxygen, nitrogen and sulphur.

**2.** Compound of formula (I) according to claim 1, wherein X represents an oxygen atom.

**3.** Compound of formula (I) according to either claim 1 or 2, wherein $R_1$ to $R_6$ and $R_8$, which may be identical or different, each represents a hydrogen atom, a hydroxy group or a linear or branched $(C_1-C_6)$alkoxy group.

**4.** Compound of formula (I) according to any one of claims 1 to 3, wherein $R_7$ represents a 2-dimethylaminoethoxy or 2-(1-pyrrolidinyl)ethoxy group.

**5.** Compound of formula (I) according to claim 1, which is (10Z)-8-(2-dimethylaminoethoxy)-2,3-dimethoxyindeno [1,2-b]indol-10(5*H*)-one O-(1-phenyl-2-propynyl)oxime, isomers thereof, and also addition salts thereof with a pharmaceutically acceptable acid.

**6.** Compound of formula (I) according to claim 1, which is (10Z)-8-(2-dimethylaminoethoxy)-2,3-dimethoxyindeno [1,2-b]indol-10(5*H*)-one O-(2-propynyl)-oxime, isomers thereof, and also addition salts thereof with a pharmaceutically acceptable acid.

**7.** Compound of formula (I) according to claim 1, which is (10Z)-8-(2-dimethylaminoethoxy)-2,3-dimethoxyindeno [1,2-b]indol-10(5*H*)-one O-(1-methyl-2-propynyl)-oxime, isomers thereof, and also addition salts thereof with a pharmaceutically acceptable acid.

**8.** Compound of formula (I) according to claim 1, which is (10Z)-8-(2-dimethylaminoethoxy)-2,3-dimethoxyindeno [1,2-b]indol-10(5H)-one O-[1-(3-furyl)-2-propynyl]oxime, isomers thereof, and also addition salts thereof with a pharmaceutically acceptable acid.

**9.** Compound of formula (I) according to claim 1, which is (10Z)-8-[2-(1-pyrrolidinyl)ethoxy]-2,3-dimethoxyindeno [1,2-b]indol-10(5*H*)-one O-[1-(3-furyl)-2-propynyl]oxime, isomers thereof, and also addition salts thereof with a pharmaceutically acceptable acid.

**10.** Compound of formula (I) according to claim 1, which is (10Z)-8-(2-dimethylaminoethoxy)-2,3-dimethoxyindeno [1,2-b]indol-10(5H)-one O-((1S)-1-methyl-2-propynyl)oxime, isomers thereof, and also addition salts thereof with a pharmaceutically acceptable acid.

**11.** Process for the preparation of compounds of formula (I) according to claim 1, **characterised in that** a compound of formula (II) :

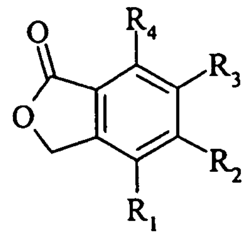

(II)

wherein $R_1$, $R_2$, $R_3$ and $R_4$ are as defined for formula (I), is reacted with N-bromosuccinimide to yield a compound of formula (III) :

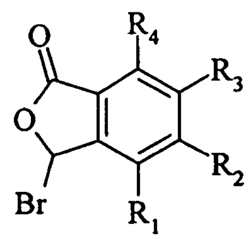

(III)

wherein $R_1$, $R_2$, $R_3$ and $R_4$ are as defined hereinbefore, which is reacted with triphenylphosphine to yield a compound of formula (IV) :

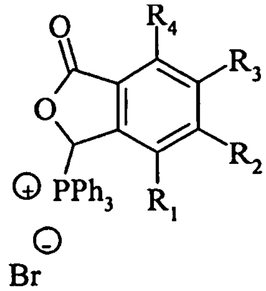

(IV)

wherein $R_1$, $R_2$, $R_3$ and $R_4$ are as defined hereinbefore, which is reacted with a compound of formula (V) :

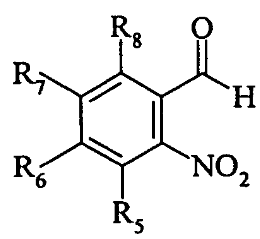

(V)

wherein $R_5$, $R_6$, $R_7$ and $R_8$ are as defined for formula (I), to yield a compound of formula (VI) :

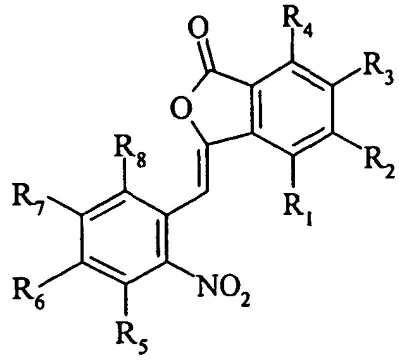

(VI)

wherein $R_1$, $R_2$, $R_3$, $R_4$, $R_5$, $R_6$, $R_7$ and $R_8$ are as defined hereinbefore, which is placed in the presence of a base to yield a compound of formula (VII) :

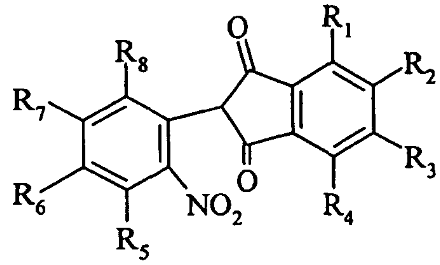

(VII)

wherein $R_1$, $R_2$, $R_3$, $R_4$, $R_5$, $R_6$, $R_7$ and $R_8$ are as defined hereinbefore, which is subjected to the action of a reducing agent to yield, after separation of isomers if desired, a compound of formula (VIII) :

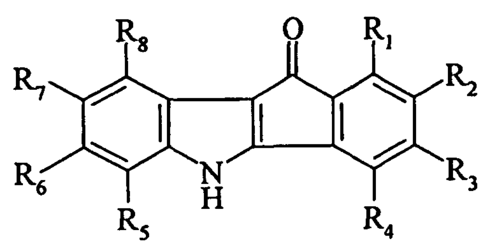

(VIII)

wherein $R_1$, $R_2$, $R_3$, $R_4$, $R_5$, $R_6$, $R_7$ and $R_8$ are as defined hereinbefore, which is reacted, if desired, with a compound of formula (IX) :

$$R'\text{-}Z \qquad (IX)$$

wherein R' represents a linear or branched ($C_1$-$C_6$)alkyl group (optionally substituted by an aryl group or by an $NR_9R_{10}$ group, wherein $R_9$ and $R_{10}$, which may be identical or different, each represents a linear or branched ($C_1$-$C_6$)alkyl group or together, with the nitrogen atom carrying them, form a nitrogen-containing heterocycle) or a linear or branched ($C_1$-$C_6$)alkenyl group, and Z represents a leaving group, such as, for example, a halogen atom or a mesylate, tosylate or trifluoromethanesulphonate group, to yield a compound of formula (X) :

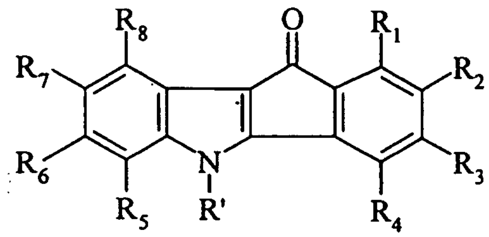

(X)

wherein $R_1$, $R_2$, $R_3$, $R_4$, $R_5$, $R_6$, $R_7$, $R_8$ and R' are as defined hereinbefore,
which compounds of formula (VIII) or (X) are reacted with a compound of formula (XI) :

$$H_2NXR_9 \qquad (XI)$$

wherein X and $R_9$ are as defined for formula (I),
to yield a compound of formula (I) which is purified, if necessary, according to a conventional purification technique, is separated, if desired, into isomers according to a conventional separation technique and is converted, if desired, into addition salts with a pharmaceutically acceptable acid or base.

**12.** Pharmaceutical composition comprising as active ingredient a compound according to any one of claims 1 to 10, alone or in combination with one or more pharmaceutically acceptable, inert, non-toxic carriers.

**13.** Pharmaceutical composition according to claim 12 for use as an anti-cancer agent.

**Patentansprüche**

**1.** Verbindungen der Formel (I):

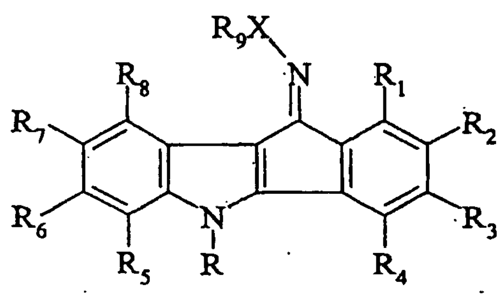

(I)

in der:

➢ R:

- * ein Wasserstoffatom,
- * eine geradkettige oder verzweigte ($C_1$-$C_6$)-Alkylgruppe, die gegebenenfalls durch eine Carboxygruppe, geradkettige oder verzweigte ($C_1$-$C_6$)-Alkoxycarbonylgruppe oder $NR_{10}R_{11}$ (worin $R_{10}$ und $R_{11}$, die gleichartig oder verschieden sind, jeweils eine geradkettige oder verzweigte ($C_1$-$C_6$)-Alkylgruppe darstellen oder gemeinsam mit dem sie tragenden Stickstoffatom einen Stickstoff-haltigen Heterocyclus bilden) substituiert ist,
- * oder eine geradkettige oder verzweigte ($C_2$-$C_6$)-Alkenylgruppe bedeutet,

➢ $R_1$ bis $R_8$, die gleichartig oder verschieden sind, jeweils:

* ein Wasserstoffatom,
* eine geradkettige oder verzweigte ($C_1$-$C_6$)-Alkylgruppe, die gegebenenfalls durch eine Arylgruppe, Carboxygruppe oder geradkettige oder verzweigte ($C_1$-$C_6$)-Alkoxycarbonylgruppe substituiert ist,
* eine Hydroxygruppe.
* eine geradkettige oder verzweigte ($C_1$-$C_6$)-Acyloxygruppe,
* eine Gruppe der Formel $NR_{12}R_{13}$, worin $R_{12}$ und $R_{13}$, die gleichartig oder verschieden sind, jeweils ein Wasserstoffatom oder eine geradkettige oder verzweigte ($C_1$-$C_6$)-Alkylgruppe bedeuten, die gegebenenfalls durch eine Gruppe der Formel $NR_{14}R_{15}$, worin $R_{14}$ und $R_{15}$, die gleichartig oder verschieden sind, jeweils eine geradkettige oder verzweigte ($C_1$-$C_6$)-Alkylgruppe bedeuten oder gemeinsam mit dem sie tragenden Stickstoffatom einen Stickstoff-haltigen Heterocyclus bilden, substituiert ist,
* eine Carboxygruppe,
* eine geradkettige oder verzweigte ($C_1$-$C_6$)-Alkoxygruppe, die gegebenenfalls durch eine Arylgruppe oder eine Gruppe der Formel $NR_{14}R_{15}$, in der $R_{14}$ und $R_{15}$, die gleich oder verschieden sind, jeweils eine geradkettige oder verzweigte ($C_1$-$C_6$)-Alkylgruppe bedeuten oder gemeinsam mit dem sie tragenden Stickstoffatom eine Stickstoff-haltigen Heterocyclus bilden, substituiert ist,
* eine geradkettige oder verzweigte ($C_2$-$C_6$)-Alkenyloxygruppe bedeuten,
* oder eine der Gruppen $R_1$ bis $R_8$ mit einer anderen angrenzenden Gruppe $R_1$ bis $R_8$ eine ($C_1$-$C_2$)-Alkylendioxygruppe bildet,

➢ X ein Sauerstoffatom oder eine Gruppe $NR_{16}$, worin $R_{16}$ ein Wasserstoffatom oder eine geradkettige oder verzweigte ($C_1$-$C_6$)-Alkylgruppe, Arylgruppe oder geradkettige oder verzweigte Aryl-($C_1$-$C_6$)-alkylgruppe darstellt, bedeutet,

➢ $R_9$ ein Wasserstoffatom oder eine Arylgruppe, Heteroarylgruppe oder geradkettige oder verzweigte ($C_1$-$C_6$)-Alkylgruppe bedeutet, mit der Maßgabe, daß die Alkylgruppe gegebenenfalls eine oder mehrere Unsättigungen aufweist und gegebenenfalls durch eine oder mehrere gleichartige oder verschiedenartige Gruppen substituiert ist, ausgewählt aus Aryl, Heteroaryl, ($C_3$-$C_8$)-Cycloalkyl, Cyano und $NR_{17}R_{18}$ (worin $R_{17}$ und $R_{18}$, die gleich oder verschieden sind, jeweils eine geradkettige oder verzweigte ($C_1$-$C_6$)-Alkylgruppe bedeuten oder gemeinsam mit dem sie tragenden Stickstoffatom einen Stickstoff-haltigen Heterocyclus bilden),

deren Isomere sowie deren Additionssalze mit einer pharmazeutisch annehmbaren Säure oder Base,
mit der Maßgabe, daß man unter Isomeren optische Isomere und geometrische Isomere der Doppelbindung C = N X $R_9$ versteht,
man unter einer Arylgruppe:

Phenyl, Biphenylyl oder Naphthyl versteht, wobei jede dieser Gruppen gegebenenfalls durch eine oder mehrere, gleichartige oder verschiedenartige Gruppen substituiert ist, ausgewählt aus Halogen, geradkettigem oder verzweigtem ($C_1$-$C_6$)-Alkyl (welches gegebenenfalls durch ein oder mehrere Halogenatome substituiert ist), geradkettigem oder verzweigtem ($C_2$-$C_6$)-Alkenyl (welches gegebenenfalls durch eine Phenylgruppe substituiert ist), geradkettigem oder verzweigtem ($C_1$-$C_6$)-Alkoxy (welches gegebenenfalls durch eine Phenylgruppe substituiert ist), Phenoxy, Nitro, Cyano, Amino (welches gegebenenfalls durch eine oder zwei geradkettige oder verzweigte ($C_1$-$C_6$)-Alkylgruppen substituiert ist) und ($C_1$-$C_2$)-Alkylendioxy,

man unter einer Heteroarylgruppe eine aromatische mono- oder bicyclische Gruppe mit 5 bis 12 Kettengliedern versteht, die ein, zwei oder drei Heteroatome ausgewählt aus Sauerstoff, Stickstoff oder Schwefel enthält, mit der Maßgabe, daß die Heteroarylgruppe gegebenenfalls durch eine oder mehrere, gleichartige oder verschiedenartige Gruppen substituiert sein kann, ausgewählt aus Halogen, geradkettigem oder verzweigtem ($C_1$-$C_6$)-Alkyl, Hydroxy, geradkettigem oder verzweigtem ($C_1$-$C_6$)-Alkoxy, geradkettigem oder verzweigtem ($C_1$-$C_6$)-Polyhalogenalkyl und Amino (welches gegebenenfalls durch eine oder mehrere geradkettige oder verzweigte ($C_1$-$C_6$)-Alkylgruppen substituiert ist),

und unter Stickstoff-haltigem Heterocyclus eine gesättigte monocyclische Gruppe mit 5 bis 7 Kettengliedern versteht, welche ein, zwei oder drei Heteroatome enthält, wobei eines dieser Heteroatome das Stickstoffatom ist und die anderen gegebenenfalls vorhandenen zusätzlichen Heteroatome aus Sauerstoff-, Stickstoff- oder Schwefelatomen ausgewählt sind.

**2.** Verbindung der Formel (I) nach Anspruch 1, worin X ein Sauerstoffatom bedeutet.

**3.** Verbindung der Formel (I) nach einem der Ansprüche 1 oder 2, worin $R_1$ bis $R_6$ und $R_8$, die gleichartig oder verschieden sind, jeweils ein Wasserstoffatom, eine Hydroxygruppe oder eine geradkettige oder verzweigte ($C_1$-$C_6$)-Alkoxygruppe bedeuten.

**4.** Verbindung der Formel (I) nach einem der Ansprüche 1 bis 3, worin $R_7$ eine 2-Dimethylaminoethoxy- oder 2-(1-Pyrrolidinyl)-ethoxy-gruppe bedeutet.

**5.** Verbindung der Formel (I) nach Anspruch 1, nämlich (10 Z)-8-(2-Dimethylaminoethoxy)-2,3-dimethoxy-indeno [1,2-b]indol-10(5*H*)-on-O-(1-phenyl-2-propinyl)-oxim, dessen Isomere sowie dessen Additionssalze mit einer pharmazeutisch annehmbaren Säure.

**6.** Verbindung der Formel (I) nach Anspruch 1, nämlich (10 Z)-8-(2-Dimethylaminoethoxy)-2,3-dimethoxy-indeno [1,2-b]indol-10(5*H*)-on-O-(2-propinyl)-oxim, dessen Isomere sowie dessen Additionssalze mit einer pharmazeutisch annehmbaren Säure.

**7.** Verbindung der Formel (I) nach Anspruch 1, nämlich (10 Z)-8-(2-Dimethylaminoethoxy)-2,3-dimethoxy-indeno [1,2-b]indol-10(5*H*)-on-O-(1-methyl-2-propinyl)-oxim, dessen Isomere sowie dessen Additionssalze mit einer pharmazeutisch annehmbaren Säure.

**8.** Verbindung der Formel (I) nach Anspruch 1, nämlich (10 Z)-8-(2-Dimethylaminoethoxy)-2,3-dimethoxy-indeno [1,2-b]indol-10(5*H*)-on-O-[1-(3-furyl)-2-propinyl]-oxim, dessen Isomere sowie dessen Additionssalze mit einer pharmazeutisch annehmbaren Säure.

**9.** Verbindung der Formel (I) nach Anspruch 1, nämlich (10 Z)-8-[2-(1-Pyrrolidinyl)-ethoxy]-2,3-dimethoxy-indeno [1,2-b]indol-10(5*H*)-on-O-[1-(3-furyl)-2-propinyl]-oxim, dessen Isomere sowie dessen Additionssalze mit einer pharmazeutisch annehmbaren Säure.

**10.** Verbindung der Formel (I) nach Anspruch 1, nämlich (10 Z)-8-(2-Dimethylaminoethoxy)-2,3-dimethoxy-indeno [1,2-b]indol-10(5*H*)-on-O-((1S)-1-methyl-2-propinyl)-oxim, dessen Isomere sowie dessen Additionssalze mit einer pharmazeutisch annehmbaren Säure.

**11.** Verfahren zur Herstellung der Verbindungen der Formel (I) nach Anspruch 1, **dadurch gekennzeichnet, daß** man eine Verbindung der Formel (II):

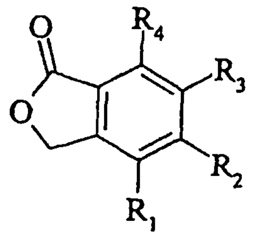

(II)

in der $R_1$, $R_2$, $R_3$ und $R_4$ die bezüglich der Formel (I) angegebenen Bedeutungen besitzen, mit N-Bromsuccinimid umsetzt zur Bildung der Verbindung der Formel (III):

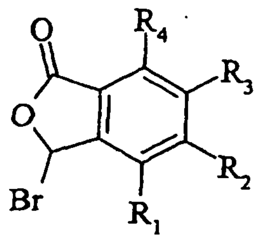

(III)

in der $R_1$, $R_2$, $R_3$ und $R_4$ die oben angegebenen Bedeutungen besitzen, welche man mit Triphenylphosphin umsetzt zur Bildung der Verbindung der Formel (IV):

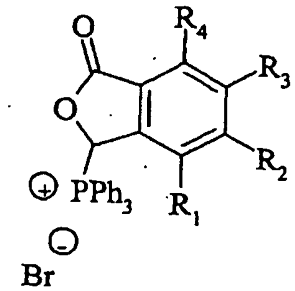

(IV)

in der $R_1$, $R_2$, $R_3$ und $R_4$ die oben angegebenen Bedeutungen besitzen, welche man mit einer Verbindung der Formel (V) umsetzt:

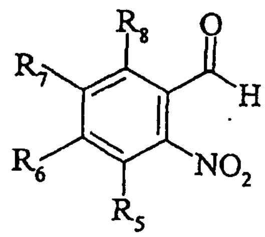

(V)

in der $R_5$, $R_6$, $R_7$ und $R_8$ die bezüglich der Formel (I) angegebenen Bedeutungen besitzen, zur Bildung der Verbindung der Formel (VI):

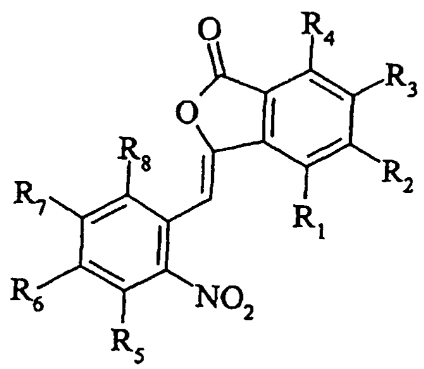

(VI)

in der $R_1$, $R_2$, $R_3$, $R_4$, $R_5$, $R_6$, $R_7$ und $R_8$ die oben angegebenen Bedeutungen besitzen, welche man mit einer Base in Kontakt bringt zur Bildung der Verbindung der Formel (VII):

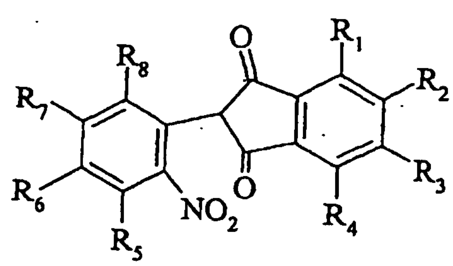

(VII)

in der $R_1$, $R_2$, $R_3$, $R_4$, $R_5$, $R_6$, $R_7$ und $R_8$ die oben angegebenen Bedeutungen besitzen, welche man mit einem Reduktionsmittel behandelt, so daß man gegebenenfalls nach der Trennung der Isomeren die Verbindung der Formel (VIII) erhält:

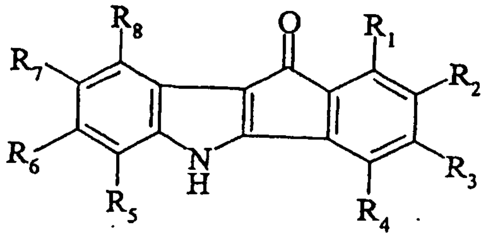

(VIII)

in der $R_1$, $R_2$, $R_3$, $R_4$, $R_5$, $R_6$, $R_7$ und $R_8$ die oben angegebenen Bedeutungen besitzen, welche man gewünschtenfalls mit einer Verbindung der Formel (IX):

$$R' - Z \qquad (IX)$$

in der R' eine geradkettige oder verzweigte ($C_1$-$C_6$)-Alkylgruppe (die gegebenenfalls durch eine Arylgruppe oder $NR_9R_{10}$ substituiert ist, in der $R_9$ und $R_{10}$, die gleichartig oder verschieden sind, jeweils eine geradkettige oder verzweigte ($C_1$-$C_6$)-Alkylgruppe bedeuten oder zusammen mit dem sie tragenden Stickstoffatom einen Stickstoff-haltigen Heterocyclus bilden) oder eine geradkettige oder verzweigte ($C_1$-$C_6$)-Alkenylgruppe darstellt und Z eine austretende Gruppe bedeutet, wie beispielsweise ein Halogenatom oder eine Mesylat-, Tosylat- oder Trifluormethansulfonatgruppe bedeutet, umsetzt,
zur Bildung der Verbindung der Formel (X):

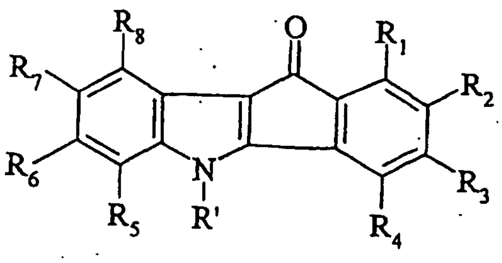

(X)

in der $R_1$, $R_2$, $R_3$, $R_4$, $R_5$, $R_6$, $R_7$, $R_8$ und R' die oben angegebenen Bedeutungen besitzen, welche Verbindungen der Formel (VIII) oder (X) man mit einer Verbindung der Formel (XI):

$$H_2NXR_9 \qquad (X)$$

in der X und $R_9$ die bezüglich der Formel (I) angegebenen Bedeutungen besitzen, umsetzt,
zur Bildung der Verbindung der Formel (I), welche man gegebenenfalls mit Hilfe einer klassischen Reinigungsmethode reinigt, welche man gewünschtenfalls mit Hilfe einer klassischen Trennmethode in die Isomeren auftrennt und welche man gewünschtenfalls in ihre Additionssalze mit einer pharmazeutisch annehmbaren Säure oder Base überführt.

**12.** Pharmazeutische Zubereitung enthaltend als Wirkstoff eine Verbindung nach einem der Ansprüche 1 bis 10 allein oder in Kombination mit einem oder mehreren inerten, nichttoxischen und pharmazeutisch annehmbaren Trägermaterialien.

**13.** Pharmazeutische Zubereitung nach Anspruch 12 als Antikrebsmittel.